# EUROPÄISCHE PATENTSCHRIFT

(11) **EP 3 791 161 B1**
(45) Veröffentlichungstag und Bekanntmachung des Hinweises auf die Patenterteilung: **28.12.2022**
(21) Anmeldenummer: 19725292.7
(22) Anmeldetag: 08.05.2019
(51) Int. Cl.: G01N 21/31, G01N 27/22, G01N 33/24, A01G 25/16, G01N 27/403, G01N 27/30, G01N 21/359

(54) **VORRICHTUNGEN UND VERFAHREN ZUR IN-SITU-BODENANALYSE**
DEVICES AND METHODS FOR IN SITU SOIL ANALYSIS
DISPOSITIFS ET PROCÉDÉS D'ANALYSE DE SOL IN SITU

(30) Priorität: 11.05.2018 DE 102018111336
(43) Veröffentlichungstag der Anmeldung: 17.03.2021
(73) Patentinhaber: STENON GMBH, 14467 Potsdam (DE)
(72) Erfinder: GRABBERT, Niels, 12489 Berlin (DE); ROTH, Dominic, 66440 Blieskastel (DE)
(74) Vertreter: Platzöder, Michael Christian
(86) Internationale Anmeldenummer: PCT/EP2019/061870
(87) Internationale Veröffentlichungsnummer: WO 2019/215257

(56) Entgegenhaltungen:
- EP-A1- 1 203 955
- CN-A- 106 950 183
- KR-A- 20160 057 140
- US-A- 5 621 669
- US-A- 5 739 536
- US-A- 5 859 536
- US-A1- 2003 009 286
- US-A1- 2016 033 437
- US-A1- 2017 299 546
- US-A1- 2018 085 003
- US-B2- 7 944 220
- US-B2- 9 285 501
- JOHN M. DELL ET AL: "MEMS-based Fabry-Perot microspectrometers for agriculture", PROCEEDINGS OF SPIE, Bd. 7319, 28. April 2009 (2009-04-28), Seite 73190K, XP055646246, 1000 20th St. Bellingham WA 98225-6705 USA ISSN: 0277-786X, DOI: 10.1117/12.819909 ISBN: 978-1-5106-2687-4

## Beschreibung

Die vorliegende Erfindung betrifft das Gebiet der Bodenanalyse, insbesondere - ohne jedoch darauf beschränkt zu sein - der technischen Analyse von landwirtschaftlich oder gartenbaulich genutzten Böden. Insbesondere betrifft die Erfindung eine Sensorvorrichtung zur In-situ-Bodenanalyse, ein Verfahren zur In-situ-Bodenanalyse und eine zur Durchführung des Bodenanalyseverfahrens eingerichtete Vorrichtung, wobei diese Vorrichtung zusammen und im Zusammenspiel mit einer oder mehreren der genannten Sensorvorrichtungen ein System zur In-situ-Bodenanalyse darstellt.

Auf dem Gebiet der Bodenanalyse werden gegenwärtig im wesentlichen laborbasierte Analyseverfahren eingesetzt, die darauf beruhen, dass einem zu analysierenden Boden eine oder mehrere Proben entnommen werden, zu einem geeigneten Labor transportiert und dort aufbereitet und analysiert werden. Anschließend wird ein entsprechender Analysebericht erstellt und einem Empfänger bzw. Auftraggeber zugestellt. Von der Probeentnahme bis zur Mitteilung des Analyseergebnisses vergehen dabei in aller Regel zumindest mehrere Tage, meistens jedoch Wochen, insbesondere während Bedarfshochzeiten wie etwa im Frühjahr (für Mitteleuropa). In einem typischen Standardlaborfür Bodenanalysen können Wassergehalt, Mikro-und Makronährstoffgehalt, elektrische Leitfähigkeit, Bodenart, pH-Wert, sowie verfügbare und Gesamtmengen bzw. Konzentrationen an Stickstoff, Phosphor und Kohlenstoff mittels standardisierter laborbasierter Analyseverfahren bestimmt werden. Eine typische Bodenprobe für einen Landwirt umfasst beispielsweise die Parameter Bodenart, Stickstoff, Phosphor-, Kalium-, Magnesium-, Bohr-, Kupfer-, Zink-, Mangan-, und Eisengehalt, sowie den pH-Wert des Bodens und möglicherweise eine Aussage zu dessen Kalkbedarf. Die bei derartigen Laboranalysen eingesetzten Methoden sind zwar sehr genau, jedoch nicht "in-situ", d. h. nicht ohne vorherige Probenentnahme und nicht vor Ort auf dem zu analysierenden Boden, beispielsweise einer landwirtschaftlichen oder gartenbaulichen Nutzfläche, einsetzbar, da entweder die dafür erforderlichen technischen Apparaturen nicht mobil sind, oder weil zur Analyse standardisierte Umweltbedingungen benötigt werden, die nur in einem Labor realisiert werden können.

Als Alternative zu Bodenanalysen im Labor stehen auch heute schon einige Verfahren zur In-situ- bzw. Semi-in-situ-Analyse von Böden zur Verfügung. Das Analysespektrum ist dabei jedoch auf die Analyse des Wassergehalts, des pH-Werts und der elektrischen Leitfähigkeit und der Bodenart des Bodens aus der Bodenprobe beschränkt. Andere Parameter, wie insbesondere die für Landwirte und Gartenbauer hochrelevanten Parameter zum Gehalt an Kalium, Magnesium, Kupfer, Mangan, Zink, Brom, Eisen, an verfügbarem Phosphor, an Humus, sowie zum Gesamtstickstoffgehalt und zum Gesamtkohlenstoffgehalt können jedoch gegenwärtig nicht in situ analysiert werden. Zudem ermöglicht keines der bislang bekannten In-situ-Analyseverfahren von sich aus eine rechtssichere Dokumentation der Messergebnisse bzw. Analyseergebnisse, wie sie Grundlage für eine Überprüfung von rechtlichen Vorschriften, etwa von gesetzlichen Düngemittelverordnungen usw., in vielen Ländern erforderlich sein können.

Aus der US 5,621,669 A ist eine Sensorsonde für Feuchtigkeit und andere Eigenschaften von Schüttgutmaterialien bekannt. Sie enthält Auswahl-, Eingabe-, Anregungs- und Isolierungsfunktionen zur Gewinnung von Signalen von einer Gruppe von Sensoren, zur Umwandlung der Signale in digitale Informationen, zum Korrelieren von Teilen der Informationen und zur Übermittlung der Informationen zu einem oder mehreren externen Aktuatoren und entfernten Empfängern und Controllern.

Aus der US 2003/0009286 A1 sind ein Gerät und ein Verfahren zur Erfassung von Bodenmerkmalen bekannt, die eingerichtet sind, eine effiziente Erfassung hochpräziser Dateninformationen über die Verteilung von Bodenmerkmalen auf einem landwirtschaftlichen Feld zu bewirken und die Dateninformationen kollektiv zu verwalten.

Aus der US 9,285,501 B2 ist ein Multi-Sensor-System zur schnellen in-situ Messung der diffusen Reflexion von Boden, der Bodenleitfähigkeit und von anderen Bodeneigenschaften in drei Dimensionen bekannt.

Aus der US 7,944,220 B2 ist ein Feuchtigkeitsgehaltssensor zur Messung des Feuchtigkeitsgehalts eines Mediums bekannt. Der Sensor enthält eine Sonde, die ein elektrisches Signal in das Medium einspeist. Komplexe Impedanzschaltungen zwischen der Sonde und der elektrischen Signalquelle ermöglichen es einer Messelektronik, ein Signal zu erzeugen, das den Feuchtigkeitsgehalt innerhalb des Mediums basierend auf Änderungen in der Permittivität des Mediums angibt.

Aus der US 5,859,536 A ist eine Messvorrichtung bekannt, aufweisend ein Paar Messelektroden, die in einem Medium angeordnet sind, und einen Schaltkreis, der mit den Messelektroden über Impedanzanpassungsnetzwerke zur Erzeugung eines Ausgangssignals, das sich als Reaktion auf eine kapazitive Änderung im Medium variiert, verbunden ist. Die Schaltung umfasst ein erstes Schaltungsteil mit den Messelektroden und ein zweites Schaltungsteil einschließlich eines Oszillators. Die ersten und zweiten Schaltungsteile werden auf ihre Impedanz abgestimmt, um eine genauere Messung der kapazitiven Änderungen zu ermöglichen.

Aus der CN 106950183 A ist ein tragbares Gerät zur Detektion von Bodennährstoffen auf Basis einer Spektral-Technologie bekannt.

Aus der US 2018/0085003 A1 ist ein Handspektrometer bekannt, das verwendet werden kann, um ein Objekt zu beleuchten und ein oder mehrerer Spektren zu messen. Die Spektraldaten des Objekts können verwendet werden, um ein oder mehrere Attribute des Objekts zu bestimmen. Insbesondere kann das Spektrometer mit einer Datenbank mit spektralen Informationen gekoppelt sein, die verwendet werden können, um bestimmen die Attribute des Objekts zu bestimmen. Das Spektrometersystem kann ein handgehaltenes Kommunikationsgerät umfassen, dass an ein Spektrometer gekoppelt ist und an dem der Benutzer Eingaben in Bezug auf das gemessene Objekt vornehmen kann.

Aus der US 2016/0033437 A1 ist eine Fernsensor-Plattform mit einem kostengünstigen Berührungssensor bekannt, der an kapazitive Platten zur Messung der Kapazität eines Bodens gekoppelt ist. Die Sensorplattform kann auch andere Sensoren zur Messung andere Gartenparameter, wie z.B. Bodenresistenz, Boden-pH-Wert, Umgebungslicht, Boden- oder Lufttemperatur und Luftfeuchtigkeit aufweisen. Basierend auf Messungen des Widerstands und der Kapazität eines Bodens kann der Feuchtigkeitsgehalt des Bodens bestimmt werden.

Aus der EP 1 203 955 A1 ist ein Bodenmessverfahren bekannt, das ein Bodenmessgerät zur Messung von Eigenschaften eines Bodens einsetzt. Das Verfahren umfasst eine Erfassung von Messdaten von einem Bodensensor basierend auf Informationen, die sich mindestens auf den Typ des Bodens einer Messstelle und dem Wassergehalt beziehen. Um Bodeneigenschaften zu berechnen, werden die erfassten Messdaten in ein Model eingegeben, das auf der Grundlage von Informationen bestimmt wird, die sich auf diese Bodenart und den Wassergehalt beziehen.

Der vorliegenden Erfindung liegt die Aufgabe zugrunde, demgegenüber verbesserte Vorrichtungen und Verfahren zur In-situ-Bodenanalyse bereitzustellen. Insbesondere ist es eine Aufgabe der Erfindung, Vorrichtungen und Verfahren zur In-situ-Bodenanalyse bereitzustellen, die es gegenüber bislang bekannten Lösungen ermöglichen, zusätzliche Bodeneigenschaften zu analysieren und/oder eine verbesserte Qualität der Analyseergebnisse zu erreichen.

Die Lösung dieser Aufgabe wird gemäß der Lehre der unabhängigen Ansprüche erreicht. Verschiedene Ausführungsformen und Weiterbildungen der Erfindung sind Gegenstand der Unteransprüche.

Ein erster Aspekt der Erfindung betrifft eine Sensorvorrichtung zur In-situ-Bodenanalyse.

Die Sensorvorrichtung weist eine Sensorbaugruppe mit einem oder mehreren Sensoren auf, die einzeln oder kumulativ zur simultanen In-situ-Messung von zumindest zwei, bevorzugt zumindest drei oder sämtlicher, der folgenden Bodeneigenschaften eines zu analysierenden Bodens und zur Bereitstellung entsprechender jeweiliger Messdaten konfiguriert sind: (a) Impedanzspektrum, (b) Temperatur, und (c) Absorptionsspektrum in einem von NIR (naher Infrarotspektralbereich) bis UV (Ultravioletter Spektralbereich) reichenden Spektralbereich, NIR-VIS-UV, und optional (d) saurer oder basischer Charakter, insbesondere pH-Wert. Dabei überschreitet der bezüglich ihrer jeweiligen Messgrößen-Aufnehmer definierte Abstand zwischen je zwei der Sensoren der Sensorbaugruppe einen Wert von 10 cm, bevorzugt von 5 cm und besonders bevorzugt von 3 cm nicht. Die Sensor-Baugruppe ist konfiguriert, zur in-situ Messung des Impedanzspektrums einen Wechselstromwiderstand eines zu vermessenden Bodenabschnitts in Abhängigkeit von der Frequenz einer an den Bodenabschnitt angelegten Messwechselspannung zu messen.

Unter einer "In-situ-Bodenanalyse" im Sinne der Erfindung ist eine Analyse eines Bodens, insbesondere eines Bodens auf einer landwirtschaftlich oder gartenbaulich genutzten Anbaufläche, zu verstehen, bei der eine Messung gewünschter Bodeneigenschaften vor Ort auf dem Boden selbst vorgenommen wird, ohne dass dazu eine Probenentnahme aus dem Boden erforderlich ist. Insbesondere kann eine In-situ-Bodenanalyse dadurch erfolgen, dass eine entsprechende Sensorvorrichtung auf oder über dem zu analysierenden Boden angeordnet, oder in diesen, zumindest teilweise, eingebracht wird, sodass die Sensorik der Sensorvorrichtung die maßgebliche Eigenschaft des Bodens messen kann, wobei dieser, zumindest im Wesentlichen, unverändert an Ort und Stelle verbleibt. Eine Auswertung der mittels einer oder mehrerer In-situ-Messungen erzeugten Messdaten zum Zwecke einer über die reine Messdatenerfassung hinausgehenden weiteren Bodenanalyse kann ebenfalls "in-situ", d.h. am Ort der Messung erfolgen, ohne dass dies jedoch zwingend ist. Im Gegensatz dazu sind Bodenanalysen, die darauf beruhen, dass zunächst dem zu analysierenden Boden eine Probe entnommen wird, die dann an gleicher oder anderer Stelle einer Messung und gegebenenfalls einer noch weiterführenden Analyse unterzogen wird, keine In-situ-Bodenanalysen im Sinne der Erfindung.

Unter einer "simultanen" In-situ-Messung von mehreren Bodeneigenschaften ist ein "in-situ"- Messvorgang zu verstehen, bei dem sich die Messzeiträume für die Messung von zumindest zwei der zu messenden Bodeneigenschaften zumindest teilweise überlappen. Insbesondere sind also Messungen mehrerer Bodeneigenschaften, die tatsächlich genau gleichzeitig stattfinden, ebenso simultane Messungen im Sinne der Erfindung, wie Messungen, bei denen beispielsweise ein erster Messzeitraum für die Messung einer ersten Bodeneigenschaft mit einem zweiten Messzeitraum für eine zweite Bodeneigenschaft zwar nicht exakt zusammenfällt, wobei es jedoch zumindest ein Zeitintervall gibt, innerhalb dessen beide Eigenschaften gleichzeitig gemessen werden. Dabei ist ein Messzeitraum für eine Bodeneigenschaft als ein Zeitraum definiert, in dem eine entsprechende Sensorik aktiv ist, um eine zugehörige Messung der Bodeneigenschaft selbst oder einer zu ihrer indirekten Bestimmung verwendeten Größe vorzunehmen.

Unter einem "Impedanzspektrum" im Sinne der Erfindung ist ein Spektrum zu verstehen, das einen Wechselstromwiderstand (Impedanz Z) eines Materials, hier eines zu vermessenden Bodenabschnitts, in Abhängigkeit von der Frequenz (ω) einer, etwa mittels Elektroden an den Bodenabschnitt, angelegten Messwechselspannung repräsentiert, was insbesondere mittels einer mathematischen Funktion Z(ω) erfolgen kann. Der Wechselstromwiderstand bei einem zweipoligen Netzwerkelement (hier der Bodenabschnitt) ist dabei als das Verhältnis von elektrischer Spannung zur Stromstärke definiert.

Unter einem Absorptionspektrum im Sinne der Erfindung, ist ein elektromagnetisches Spektrum zu verstehen, das "dunkle" Spektrallinien, also Einschnitte im spektralen Verlauf, enthält, die entstehen, wenn breitbandige elektromagnetische Strahlung Materie be- oder durchstrahlt und Strahlungsquanten (Photonen) bestimmter Wellenlängen oder Wellenlängenbereiche dabei von der Materie absorbiert werden. Dabei können einer oder mehrere verschiedene Absorptionsmechanismen, meist wellenlängenabhängig, auftreten. Insbesondere sind elektronische Übergänge zwischen verschiedenen Energieniveaus von Atomen, Molekülen oder Kristallen oder anderen Festkörpern (beispielsweise im Rahmen von Lumineszenz), sowie Anregungen von anderen Freiheitsgraden, insbesondere von Rotations- oder Schwingungsfreiheitsgraden von Molekülen und in Festkörpern möglich. Mittels Vergleich von gewonnenen Absorptionsspektren, insbesondere Reflexionsspektren, mit entsprechenden Referenzspektren lassen sich so qualitative und/oder quantitative Rückschlüsse auf die Materialzusammensetzung der vermessenen Materie ziehen.

Unter einem "Messgrößen-Aufnehmer" oder kurz "Aufnehmer" im Sinne der Erfindung ist der Teil einer Messeinrichtung, d.h. eines Sensors, zu verstehen, der auf eine Messgröße unmittelbar anspricht. Damit ist der Aufnehmer das erste Element einer Messkette. Insbesondere kann der Aufnehmer - ohne darauf beschränkt zu sein - in Form einer oder mehrerer Elektroden, eines optischen Empfängers, oder eines Temperatur-Messfühlers implementiert sein. Als Abstand zwischen zwei Aufnehmern ist die kürzeste Entfernung zwischen beiden zu verstehen.

Die Sensorvorrichtung gemäß dem ersten Aspekt der Erfindung zeichnet sich dadurch aus, dass sie zum einen mindestens zwei verschiedene Bodeneigenschaften sensorisch und, zumindest im Wesentlichen, nicht-destruktiv erfassen kann, die noch dazu so ausgewählt sind, dass zwischen Ihnen jeweils eine deutliche Korrelation existiert, die es ermöglicht aus den mittels Messung gewonnenen Messdaten mittels Datenfusion eine gegenüber den Einzelmessungen erhöhte Messgenauigkeit und somit eine erhöhte Bodenanalysequalität zu erreichen. Zudem sind die Messgrößen-Aufnehmer der Sensoren auf sehr engem Raum konzentriert (z.B. auf einer Fläche ≤ 100 cm², bevorzugt ≤ 25 cm², besonders bevorzugt ≤ 9 cm²), sodass der vermessene Bodenabschnitt in guter Näherung als homogen angenommen werden kann, was zur weiteren Verbesserung der Messgenauigkeit genutzt wird, insbesondere im Hinblick darauf, dass die Korrelation zwischen den einzelnen Messergebnissen stark abstandsabhängig ist und regelmäßig nur bei geringen Abständen eine signifikante Verbesserung der Bodenanalysequalität mittels Datenfusion ermöglicht.

Zudem finden die Messungen simultan statt, sodass sich zeitabhängige Messfehler, minimieren lassen. Ein solcher Messfehler könnte andernfalls beispielsweise auftreten, falls eine Impedanzmessung zu einer lokalen Erwärmung des Bodens führte, die dann bei einer zeitlich versetzten nachfolgenden Temperaturmessung zu verfälschten Temperaturmesswerten führen würde. Zudem ermöglicht es die Kombination der genannten verschiedenen Messverfahren, über bisherige Möglichkeiten zur In-situ-Messung hinausgehende Bodeneigenschaften mittels Kombination der Messdaten der einzelnen Messungen zu erreichen. Auch verkürzt eine simultane Messung gegenüber rein sequenziellen Einzelmessungen den erforderlichen Gesamtzeitaufwand für den Messvorgang.

Da das Entnehmen von Bodenproben ebenso entfällt, wie deren Verbringen in ein ex-situ Labor, können die Bodenanalyseergebnisse in kürzester Zeit, insbesondere auch vor Ort unmittelbar bei der Messung bereitgestellt werden, sodass kein nennenswerter zeitlicher Verzug mehr bis Zurverfügungstellung der Analyseergebnisse erforderlich ist.

Nachfolgend werden bevorzugte Ausführungsformen der Sensorvorrichtung beschrieben, die jeweils, soweit dies nicht ausdrücklich ausgeschlossen wird oder technisch unmöglich ist, beliebig miteinander sowie mit den weiteren beschriebenen anderen Aspekten der Erfindung kombiniert werden können.

Bei einigen Ausführungsformen enthält die Sensorbaugruppe zur In-situ-Erfassung eines Impedanzspektrums des zu analysierenden Bodens einen Impedanzsensor. Dieser weist auf (i) ein erstes Trägerelement; (ii) zwei auf dem ersten Trägerelement aber von diesem und untereinander elektrisch isoliert angeordnete, Leiterbahnen, von denen zumindest eine einen elektrisch leitfähigen korrosionsbeständigen Polymer- oder Kompositwerkstoff enthält; (iii) und eine Steuerungsvorrichtung. Die Steuerungsvorrichtung ist konfiguriert, zwischen den beiden Leiterbahnen eine Wechselspannung anzulegen, deren Frequenz über einen vorbestimmten Frequenzbereich hinweg zu variieren, und dabei im Betrieb der Sensorvorrichtung, wenn diese so in den zu analysierenden Boden eingebracht ist, dass die Leiterbahnen mit diesem in elektrischen Kontakt stehen, ein Impedanzspektrum des zu analysierenden Bodens in Reaktion auf die an ihn über die Leiterbahnen angelegte Wechselspannung zu erfassen und in Form entsprechender Messdaten bereitzustellen. Auf diese Weise ist die Sensorvorrichtung in der Lage, ein Impedanzspektrum des zu analysierenden Bodens aufzunehmen, unter dessen Zuhilfenahme insbesondere verschiedene Bodenarten, Bodentexturen, Leitfähigkeiten, Wassergehalt, lonenkonzentrationen und lonenarten bestimmt werden können.

Die besondere Gestaltung der Leiterbahnen auf dem Trägerelement, sowie deren spezielle Materialwahl ermöglichen sowohl einen besonders guten elektrischen Kontakt zum umgebenden Boden, als auch eine hohe Beständigkeit, insbesondere Abriebs-und Korrosionsbeständigkeit, gegenüber dem Boden und somit eine lange Lebensdauer der Sensorvorrichtung.

Die Leiterbahnen können insbesondere auf dem ersten Trägerelement aufgewickelt sein, bevorzugt so, dass die beiden Leiterbahnen parallel zueinander verlaufen, was eine besonders genaue und platzoptimierte Lösung darstellt. Unter "elektrischer Leitfähigkeit" ist dabei eine physikalische Größe zu verstehen, die angibt, wie stark die Fähigkeit eines Stoffes ist, den elektrischen Strom zu leiten. Unter "elektrisch leitfähig" im Sinne der Erfindung ist demnach eine elektrische Leitfähigkeit zu verstehen, die (bei 25 °C) mindestens 106 S/m beträgt, also zumindest der Leitfähigkeit von Metallen entspricht.

Bei einigen weiteren Ausführungsformen ist das erste Trägerelement zumindest in einem von den Leiterbahnen überdeckten Bereich elektrisch leitfähig, insbesondere metallisch, und die Steuerungsvorrichtung ist des Weiteren konfiguriert, während der Erfassung des Impedanzspektrums des zu analysierenden Bodens das elektrische Potential dieses zumindest einen Bereichs auf ein Massepotential zu legen. Auf diese Weise kann eine Signalverfälschung des aufgenommenen Impedanzspektrums durch externe elektromagnetische Einkopplungen verringert, oder sogar vermieden werden. Das Massepotential kann dabei insbesondere das Massepotential (Nullpotential) einer Stromversorgung der Sensoreinrichtung, beispielsweise eines dazu eingesetzten Akkus, sein.

Bei einigen weiteren Ausführungsformen schließt der vorbestimmte Frequenzbereich den Bereich von 100 Hz bis 1 MHz ein, wodurch sich ein Spektrum ermitteln lässt, das aufgrund seiner Breite und Lage im elektromagnetischen Spektrum besonders gut Rückschlüsse auf eine Vielzahl verschiedener Bodeneigenschaften ermöglicht.

Bei einigen weiteren Ausführungsformen ist das erste Trägerelement als zumindest abschnittsweise hohler Dorn zum zumindest abschnittsweisen Einbringen in den zu analysierenden Boden ausgebildet. Zudem ist auf der Oberfläche des Dorns eine Isolationsschicht aufgebracht, auf der wiederum die beiden Leiterbahnen angeordnet, insbesondere aufgewickelt, sind. Die Steuerungsvorrichtung ist dabei im Inneren eines hohlen Abschnitts des ersten Trägerelements angeordnet. Die dornartige Ausbildung des ersten Trägerelements dient dazu, zumindest teilweise in den zu analysierenden Boden eingebracht (eingestochen) zu werden, und dabei die als Messgrößen-Aufnehmer des Impedanzsensors dienenden Leiterbahnen in Kontakt mit dem Boden zu bringen. Mittels der Isolation sind die Leiterbahnen elektrisch untereinander und von dem Dorn entkoppelt, der-wie vorausgehend beschrieben - insbesondere auf Massepotential gelegt sein kann. Zudem ist die Steuerungsvorrichtung im Inneren des hohlen Abschnitts des ersten Trägerelements vor unerwünschten Einwirkungen, insbesondere des Bodens oder der sonstigen Umgebung geschützt, insbesondere gegen Staub, Feuchtigkeit und korrosionsverursachende Stoffe.

Bei einigen weiteren Ausführungsformen enthält die Sensorbaugruppe zur Erfassung einer Temperatur des zu analysierenden Bodens einen Temperatursensor, wobei dieser zusammen mit dem Impedanzsensor als integrierte Impedanz/Temperatur-Sensorbaugruppe ausgebildet ist, die konfiguriert ist, simultan und in-situ sowohl ein Impedanzspektrum als auch eine Temperatur des zu analysierenden Bodens zu erfassen und jeweils in Form entsprechender Messdaten bereitzustellen. Auf diese Weise werden nicht nur zumindest zwei verschiedene Messgrößen ermittelt, die wie vorausgehend erläutert, ein breiteres Spektrum an bestimmbaren Bodeneigenschaften, sowie eine höhere Analysequalität ermöglichen, sondern es wird ebenso eine besonders hohe Integrationsdichte ermöglicht, die es erlaubt, die Sensorvorrichtung besonders platzsparend auszubilden.

Der Temperatursensor, oder Teile davon, kann bzw. können insbesondere ebenso wie die Steuerungsvorrichtung im Inneren eines hohen Abschnitts des ersten Trägerelements angeordnet sein, um wie diese dort vor unerwünschten externen Einflüssen geschützt zu sein.

Das erste Trägerelement und/oder zumindest eine der Leiterbahnen können dabei insbesondere zugleich als Temperaturmesssonde (d.h. Messgrößen-Aufnehmer) dienen und dazu wärmeleitend an den Temperatursensor angeschlossen sein.

Bevorzugt ist das erste Trägerelement bzw. die zumindest eine Leiterbahn deshalb mittels eines gut wärmeleitenden Materials, insbesondere eines Metalls, wie etwa Aluminium oder einem gut wärmeleitenden Polymer- oder Kompositmaterial, ausgebildet.

Bei einigen Ausführungsformen ist der Temperatursensor in die Steuerungsvorrichtung integriert, etwa auf einem gemeinsamen PCB oder einer gemeinsamen integrierten Schaltung, was wiederum im Sinne einer hohen und daher platzsparenden Integration der Sensorvorrichtung vorteilhaft ist, insbesondere auch im Hinblick auf die Erreichung einer möglichst in ihrer Dichte optimierten Anordnung der Messgrößen-Aufnehmer der verschiedenen Sensoren der Sensorvorrichtung.

Bei einigen Ausführungsformen ist der Temperatursensor im Inneren eines elektrisch leitfähigen Abschnitts des ersten Trägerelements angeordnet, sodass sich eine zumindest teilweise Abschirmung des Temperatursensors vor etwaigen durch die Leiterbahnen beim Anlegen der Wechselspannung daran erzeugten elektromagnetischen Wechselwirkungen ergibt, wodurch die Messgenauigkeit erhöht und unerwünschten Störeffekten begegnet werden kann.

Bei einigen Ausführungsformen enthält die Sensorbaugruppe zur In-situ-Erfassung eines Absorptionsspektrums des zu analysierenden Bodens eine Absorptionsspektrometerbaugruppe. Diese weist zumindest zwei, insbesondere auf Basis eines Fabry-Perot Interferometers aufgebaute, MEMS-Absorptionsspektrometer, (d.h. Absorptionsspektrometer, welche zumindest teilweise mittels MEMS-Technologie hergestellt wurden, insbesondere MEMS-Komponenten enthalten) auf, deren spektrale Abdeckung sich zumindest für Teilbereiche des elektromagnetischen Spektrums unterscheidet, wobei kumulativ durch die Gesamtheit der MEMS-Absorptionsspektrometer ein Absorptionsspektrum des zu analysierenden Bodens erfassbar ist, welches sowohl Anteile im NIR-Bereich als auch im VIS-Bereich als auch im UV Bereich aufweist. Die spektrale Abdeckung kann insbesondere durchgängig vom NIR-Bereich bis zum UV-Bereich reichen und insbesondere den Bereich von 350nm bis 1700nm einschließen, um eine besonders stark differenzierende Messung ein einem für die Bodenanalyse regelmäßig besonders relevanten Spektralbereich zu ermöglichen.

Bei einigen Ausführungsformen weist die Absorptionsspektrometerbaugruppe des Weiteren einen beweglichen, insbesondere rotierbaren und/oder translatorisch verschiebbaren, Träger auf, auf dem die Absorptionsspektrometer so angeordnet sind, dass sie bei einer Bewegung des Trägers relativ zu einer virtuellen Messfläche, an der im Messbetrieb der Sensorvorrichtung der zu analysierende Boden zu liegen kommt, einen dabei von den Absorptionsspektrometern abzutastenden Bereich des Bodens spektrometrisch vermessen können, um ein über den abzutastenden Bereich integriertes Absorptionsspektrum zu erfassen. Auf diese Weise lassen sich statistisch besser verwertbare und genauere Ergebnisse erzielen, wobei eine möglichst große Bodenfläche, idealerweise bei möglichst geringem Abstand, gescannt werden kann. Im Falle eines rotierbaren Trägers, kann das gemessene Absorptionsspektrum insbesondere über den Drehwinkel des Trägers integriert oder gemittelt werden, und im Falle einer translatorischen Bewegung insbesondere über die Wegstrecke dieser Translationsbewegung. Auf diese Weise haben nichtspezifische Charakteristika des Bodens, beispielsweise kleine Steine, Zweige usw., im Mittel nur einen reduzierten, insbesondere geringen Einfluss auf die erzielten Messergebnisse, der noch dazu, insbesondere mittels gezielter Filterung, beispielsweise mittels Schwellwerten, zumindest weitgehend beseitigt kann.

Bei einigen Ausführungsformen ist auf dem beweglichen Träger des Weiteren zumindest eine Quelle für elektromagnetische Strahlung angeordnet, die konfiguriert ist, im Messbetrieb während der Bewegung des Trägers relativ zu der Messfläche den dabei von den Absorptionsspektrometern abzutastenden Bereich des Bodens zur Erzeugung des zu messenden Absorptionsspektrums elektromagnetisch zu bestrahlen. Auf diese Weise ist es möglich, einerseits aufgrund der Bewegung eine vergrößerte Bodenfläche abzuscannen, andererseits jedoch die relative Positionierung der Strahlungsquelle zu den Absorptionsspektrometern unverändert zu lassen, was insbesondere eine erhöhte Messgenauigkeit zur Folge haben kann und Justageaufwand verringern bzw. vermeiden helfen kann.

Bei einigen Ausführungsformen weist die Absorptionsspektrometerbaugruppe des Weiteren eine bewegliche Shuttervorrichtung auf. Diese ist konfiguriert, temporär eine Blende in einen zwischen den Absorptionsspektrometern und der Messfläche definierten Raumbereich einzufahren, wobei auf der den Absorptionsspektrometern zugewandten Seite der Blende eine Kalibrierreferenz, wie etwa insbesondere Spectralon, zur Kalibrierung zumindest eines, bevorzugt sämtlicher, der Absorptionsspektrometer angeordnet ist. Dadurch kann sich die Sensorvorrichtung automatisch, beispielsweise nach einer gewissen vorbestimmten Anzahl von Messvorgängen, selbst kalibrieren (etwa mittels Dark Current- und Referenzkalibrierung), insbesondere auch im Rahmen der In-situ-Bodenanalyse selbst.

Bei einigen Ausführungsformen weist die Absorptionsspektrometerbaugruppe des Weiteren eine in einem dem aufzunehmenden Absorptionsspektrum entsprechenden Wellenlängenbereich, zumindest im Wesentlichen, transparente Optik auf, die in dem Raumbereich zwischen den Absorptionsspektrometern und der Messfläche angeordnet ist, um diese räumlich voneinander abzutrennen. Dabei ist die Optik auf ihrer der Messfläche zugewandten Seite mit einer hydrophilen Nanobeschichtung versehen, die insbesondere auch eine gegenüber dem Körpermaterial der Optik erhöhte Kratzfestigkeit aufweisen kann. Die Optik kann im Hinblick auf die Erreichung einer möglichst hohen Kratzfestigkeit insbesondere auch aus Saphirglas ausgebildet sein. Die räumliche Trennung dient insbesondere dem Schutz der Absorptionsspektrometer sowie gegebenenfalls der Shuttervorrichtung gegenüber unerwünschten externen Einwirkungen (insbesondere gegen Staub, Feuchtigkeit, mechanische Einwirkung), etwa durch den zu analysierenden Boden.

Bei einigen Ausführungsformen enthält die Sensorbaugruppe zur In-situ-Erfassung eines sauren oder basischen Charakters, insbesondere eines pH-Werts, des zu analysierenden Bodens eine Potentialmessbaugruppe. Diese weist auf: (i) ein zweites Trägerelement, (ii) eine in oder an dem zweiten Trägerelement angeordnete Elektrolyt/Metall-Referenzelektrode; (iii) eine an einer beim Messbetrieb zur Kontaktierung des zu analysierenden Bodens vorgesehenen Oberfläche des zweiten Trägerelements angeordnete Metalloxidelektrode; (iv) ein am zweiten Trägerelement zwischen der Metalloxidelektrode und der Elektrolyt/Metall-Referenzelektrode angeordnetes und mit der Elektrolyt/Metall-Referenzelektrode in Kontakt stehendes lonendiaphragma; (v) eine an der zur Kontaktierung des zu analysierenden Bodens vorgesehenen Oberfläche des zweiten Trägerelements angeordnete und von der Metalloxidelektrode elektrisch isolierte korrosionsbeständige Kalibrierelektrode; und(vi)eine Messeinrichtung. Dabei ist die Messeinrichtung konfiguriert: (a) zur Bestimmung eines aktuellen Zustands der Metalloxidelektrode einen zwischen der Kalibrierelektrode und der Metalloxidelektrode auftretenden elektrischen Widerstand und/oder eine dazwischen auftretende elektrische Kapazität zu messen, wenn diese beiden Elektroden jeweils mit dem zu analysierenden Boden in Kontakt stehen; und (b) zur Bestimmung eines sauren oder basischen Charakters, insbesondere eines pH-Werts, des zu analysierenden Bodens eine zwischen der Referenzelektrode und der Metalloxidelektrode auftretende elektrische Potentialdifferenz unter Berücksichtigung einer zuvor auf Basis des bestimmten aktuellen Zustands der Metalloxidelektrode festgelegten Messkalibrierung zu messen, wenn diese beiden Elektroden jeweils mit dem zu analysierenden Boden in Kontakt stehen.

Die Messung des sauren oder basischen Charakters des Bodens durch die Potentialmessbaugruppe kann entsprechend im Betrieb so erfolgen, dass gemäß dem Teilmerkmal (b) oben eine zwischen der Referenzelektrode unter Metalloxidelektrode auftretende elektrische Potentialdifferenz gemessen wird. Diese Potentialdifferenz ist vom sauren bzw. basischen Charakter des Bodens, der mit den beiden Elektroden während des Messvorgangs im Berührungskontakt steht, abhängig, sodass sie zu dessen Messung genutzt werden kann. Das gemessene Potential entspricht oder korrespondiert zumindest zu dem RedOx-Potential zwischen den beiden Elektroden, wobei die zugehörige chemische RedOx-Gleichung wie folgt lautet:

*RedOx* xMe + yH₂O ↔ MeₓO_{y} + y2H⁺+ y2e⁻

Dabei steht die Abkürzung "Me" für ein Metall. Die Potentialdifferenz beruht somit auf den besonderen elektrochemischen Eigenschaften von Metalloxid/Metall-basierten Sensoren, insbesondere pH-Sensoren, wobei das Metalloxid/Metall-System insbesondere Sb₂O₃/Sb, IrO₂/IR, TIO₂/TI oder RuO₂/Ru sein kann. Diese Materialien weisen eine direkte Oxidations- bzw. Reduktionsabhängigkeit bei gleichzeitig guter elektrischer Leitfähigkeit im Verhältnis zu der sie umgebenden Wasserstoffionenkonzentration (pH-Wert) im Boden auf. Ihr Redox-Potential kann somit zu der Referenzelektrode korreliert und der saure oder basische Charakter bzw. pH-Wert des Bodens daraus bestimmt werden. Zudem ist das Material der Metalloxidelektrode bevorzugt so gewählt, dass es über gute Abriebs und Stoßfestigkeiten (gegenüber dem Boden) verfügt, was bei den genannten Materialsystemen der Fall ist.

Die Redox-Potentialdifferenz wird mittels Messung der zwischen den beiden Elektroden durch das lonendiaphragma fließenden lonenströme ermittelt, wobei vorzugsweise zusätzlich ein Impedanzwandler bzw. Verstärker vorgesehen ist, um die möglicherweise nur sehr schwachen Ströme vor ihrer Messung zum Zwecke einer erhöhten Messbarkeit und Messgenauigkeit zu wandeln bzw. zu verstärken. Auch die Größe des lonendiaphragmas ist bevorzugt relativ zur Größe des (zweiten) Trägerelements möglichst groß gewählt, um für den lonenstromfluss durch das lonendiaphragma eine möglichst große Querschnittsfläche zu bereitzustellen.

Allerdings sind Metalloxide in aller Regel nur begrenzt korrosionsbeständig gegenüber Säuren bzw. Basen, sodass Metalloxidelektroden beim Einsatz zur Bodenanalyse oftmals mit der Zeit degradieren, was insbesondere zur Reduktion der Schichtdicke der Metalloxidelektrode mit einer daraus folgenden Veränderung des elektrischen Widerstands, somit der Stromstärke und somit wiederum der Messergebnisse führen kann. Daher ist die Messeinrichtung gemäß Teilmerkmal (a) des Weiteren konfiguriert, einen aktuellen Zustand, insbesondere eine aktuelle Schichtdicke, der Metalloxidelektrode zu bestimmen, indem sie einen zwischen der Kalibrierelektrode und der Metalloxidelektrode auftretenden elektrischen Widerstand (oder Leitfähigkeit) und/oder eine dazwischen auftretende elektrische Kapazität misst, während die beiden jeweils mit dem zu analysierenden Boden in Kontakt stehen, der dann beide Elektroden elektrisch verbindet. Die Messung kann insbesondere zyklisch erfolgen. Die Leitfähigkeit und/oder Kapazität des Bodens kann insbesondere, soweit nicht a priori bekannt, mittels des vorausgehend genannten Impedanzsensors der Sensorvorrichtung bestimmt werden, sodass entsprechend die Leitfähigkeit bzw. der elektrische Widerstand oder die Kapazität der Metalloxidschicht durch die Messeinrichtung mittels der o.g. Messung ermittelt werden kann, wobei die Leitfähigkeit bzw. Kapazität der Metalloxidelektrode direkt mit ihrer Metalloxid-Schichtdicke korreliert. Somit kann die Messung mittels der Messeinrichtung auf Basis der Messung des Zustands der Metalloxidelektrode bei Bedarf, insbesondere auch vorbeugend zyklisch, neu kalibriert werden, um die Messgenauigkeit auch über lange Zeiträume hinweg trotz auftretender Degradation des Metalloxids sicherzustellen.

Bei einigen Ausführungsformen ist die Kalibrierelektrode aus einem Material gefertigt, das einen elektrisch leitfähigen und korrosionsbeständigen Polymer- und/oder Kompositwerkstoff enthält. Diese Werkstoffe können insbesondere ein geringes Gewicht, eine hohe Korrosionsbeständigkeit sowie eine lange Haltbarkeit und Stabilität als Kalibrierungsreferenz als Vorteile mit sich bringen.

Bei einigen Ausführungsformen ist das zweite Trägerelement als Dorn zum zumindest abschnittsweisen Einbringen in den zu analysierenden Boden ausgebildet, wobei auf der Oberfläche des Dorns eine Isolationsschicht aufgebracht ist, auf der die Metalloxidelektrode, das lonendiaphragma, und/oder die Kalibrierelektrode angeordnet sind. Dies erlaubt eine besonders kompakte Implementierung. Zudem kann die Elektrolyt/Metall-Referenzelektrode vorteilhaft innerhalb des (zweiten) Trägerelements, also des Dorns, angeordnet und somit gegen unerwünschte externe Einwirkungen geschützt sein.

Bei einigen Ausführungsformen weist die Sensorvorrichtung des Weiteren eine Kommunikationsvorrichtung zur Übermittlung von erfassten Messdaten zu deren Auswertung an eine bezüglich der Sensorvorrichtung externe Gegenseite auf. Die Gegenseite kann insbesondere eine separate Auswertevorrichtung oder eine entfernte (Remote) Rechenplattform, beispielsweise in einer Cloud-Umgebung, oder ein Backend-Server oder ein verteiltes Rechnernetzwerk sein. Auf diese Weise lässt sich die Weiterverarbeitung der Messdaten, zur Bestimmung der finalen Bodenanalyseergebnisse aus der Sensorvorrichtung auslagern, was insbesondere dann zweckmäßig sein kann, wenn komplexe, aufwendige Berechnungen erforderlich sind, die schneller oder besser auf zentralen oder spezialisierten Rechenanlagen ausführbar sind als lokal an der Sensorvorrichtung selbst.

Allerdings ist es gleichermaßen bei anderen Ausführungsformen möglich, die zur Auswertung der Messergebnisse erforderliche Ausrüstung in der Sensorvorrichtung selbst vorzusehen. Doch auch in diesem Fall kann es zweckmäßig sein, die genannte Kommunikationsvorrichtung in der Sensorvorrichtung vorzusehen, jedenfalls um Remote-Updates von zur Auswertung und/oder zur Steuerung der Sensorvorrichtung verwendeter Software zu ermöglichen.

Bei einigen Ausführungsformen ist die Kommunikationsvorrichtung konfiguriert, die Messdaten drahtlos mittels einer Kommunikation auf Basis der LoRa-Funktechnologie und/oder der NarrowBand-Internet of Things, NB-loT,-Funktechnologie zu übermitteln. Diese Technologien sind insbesondere dann besonders vorteilhaft, wenn die Sensorvorrichtung an Orten zum Einsatz kommen soll, an denen eine sonstige Datenfunkabdeckung, beispielsweise über herkömmlichen Mobilfunk, fehlt oder ungenügend ausgebildet ist. Die genannten Funktechnologien erlauben eine drahtlose Datenübermittlung über Distanzen von bis zu 30 km, was ungefähr dem Doppelten der maximalen Reichweite (Endgerät - Basisstation) herkömmlicher Mobilfunktechnologien entspricht. Dabei ist der Energieverbrauch typischerweise sehr gering, sodass diese Technologien insbesondere auch in mobilen, batteriebetriebenen Geräten sinnvoll einsetzbar sind. Zudem ist zumindest die Nutzung der LoRa-Technologie in vielen Ländern lizenzfrei möglich, was sich entsprechend positiv auf die Betriebskosten auswirkt.

Die Kommunikationsvorrichtung kann insbesondere auch konfiguriert sein, Daten zu empfangen, insbesondere geräteextern bestimmte Bodenanalyseergebnisdaten, sodass entsprechende Informationen an der Sensorvorrichtung selbst an einer geeigneten Mensch-Maschine-Schnittstelle, beispielsweise einer Anzeigevorrichtung oder einer optischen oder akustischen Ausgabevorrichtung dem Benutzer in situ zur Verfügung gestellt werden können.

Bei einigen Ausführungsformen weist die Sensorvorrichtung des Weiteren eine sichere Speichervorrichtung zur gegen unautorisierten Zugriff geschützten Ablage einer eindeutigen Geräteidentifikation der Sensorvorrichtung und/oder zumindest eines kryptographischen Schlüssels zur Verschlüsselung von mittels der Kommunikationsvorrichtung übermittelten Mess- und/oder Metadaten auf. Die Metadaten können insbesondere - ohne darauf beschränkt zu sein - einen Ort, einen Zeitpunkt und/oder einen Messmodus einer in-situ mit der Sensorvorrichtung vorgenommenen Messung sowie die Geräteidentifikation oder eine Benutzeridentifikation repräsentieren. Auf diese Weise lassen sich insbesondere eine, insbesondere gegenüber "man-in-the-middle" Attacken geschützte Kommunikation über die Kommunikationsvorrichtung, sowie eine gegen unautorisierte Veränderung geschützte Geräteidentität realisieren.

Bei einigen Ausführungsformen ist die Kommunikationsvorrichtung des Weiteren konfiguriert, zu übermittelnde Mess- und/oder Metadaten in eine als externe Gegenseite auftretende Blockchain zu schreiben oder eine andere externe Gegenseite dazu zu veranlassen, die an sie übermittelten Mess- und/oder Metadaten in eine Blockchain zu schreiben. Diese Ausführungsformen sind insbesondere im Hinblick auf eine rechtssichere Dokumentation der Messergebnisse vorteilhaft. Zudem erlauben auch diese Ausführungsformen einen Schutz der Kommunikation, insbesondere im Hinblick auf einen Schutz gegen nachträgliche Verfälschung der gewonnenen Messergebnisse beziehungsweise Bodenanalyseergebnisse.

Bei einigen Ausführungsformen ist die Sensorvorrichtung konfiguriert, eine Authentifizierung eines Nutzers der Sensorvorrichtung vorzunehmen und ein Übermitteln der Mess- und/oder Metadaten an eine externe Gegenseite nur dann zuzulassen, wenn die Authentifizierung erfolgreich verlaufen ist. Auch mit dieser Maßnahme können die Kommunikation und Dokumentation der Messergebnisse gegenüber Angriffen, insbesondere im Hinblick auf eine Verfälschung der Messdaten, geschützt werden. Durch eine oder mehrere der vorgenannten Schutzmaßnahmen lassen sich somit die Voraussetzungen für eine Erreichung einer, gegebenenfalls gesetzlich vorgeschriebenen, rechtssicheren Dokumentation der Messergebnisse erzielen.

Bei einigen Ausführungsformen weist die Sensorvorrichtung des Weiteren eine Positionsbestimmungsvorrichtung zur Bestimmung einer aktuellen Position der Sensorvorrichtung und zur Bereitstellung entsprechender die Position kennzeichnender Metadaten auf. Dies erlaubt es insbesondere, zusammen mit den Messdaten auch einen Ort der Messung mittels entsprechender Metadaten zur Verfügung zu stellen. Außerdem kann so eine räumliche Überwachung der Sensorvorrichtung implementiert werden, was ebenfalls einen zusätzlichen Schutz gegen über Missbrauch, insbesondere eines Missbrauchs durch nicht autorisierte Personen, ermöglicht.

Bei einigen Ausführungsformen ist die Sensorvorrichtung als tragbare Einheit ausgebildet. Dies bedeutet insbesondere, dass die Dimensionen und das Gewicht der Vorrichtung ein einfaches Tragen einen menschlichen Benutzer, beispielsweise zu einer Messstelle auf einem Ackerboden, ohne weiteres erlaubt. Idealerweise betragen daher die Dimensionen der Sensorvorrichtung in jeder Richtung maximal wenige Dezimeter, z.B. < 50 cm) und das Gewicht liegt bevorzugt unterhalb von 25 kg, idealerweise unterhalb von 10 kg. Somit kann die Sensorvorrichtung besonders flexibel, und ohne Zuhilfenahme von Fahrzeugen oder anderen Manövriervorrichtungen eingesetzt werden.

Ein zweiter Aspekt der Erfindung betrifft ein computer-implementiertes Verfahren zur Bodenanalyse, aufweisend:
(i) Empfangen von Messdaten bezüglich von zumindest zwei, bevorzugt zumindest drei oder sämtlicher, der folgenden Bodeneigenschaften eines zu analysierenden Bodens: (a) Impedanzspektrum, (b) Temperatur, (c) Absorptionsspektrum in einem von NIR bis UV reichenden Spektralbereich NIR-VIS-UV, und optional (d) saurer oder basischer Charakter, insbesondere pH-Wert; und (ii) Bestimmen von zumindest einer der Bodeneigenschaften oder zumindest einer daraus abgeleiteten Bodeneigenschaft auf Basis einer Verknüpfung der empfangenen Messdaten mittels Datenfusion zur Gewinnung eines jeweiligen Messergebnisses für die zumindest eine zu bestimmende Bodeneigenschaft. Mithilfe dieses Verfahrens wird es somit möglich, die Messergebnisse bezüglich der genannten Bodeneigenschaften im Rahmen einer Datenfusion miteinander zu verknüpfen, wobei wiederum darauf hinzuweisen ist, dass die genannten Bodeneigenschaften so ausgewählt sind, dass zwischen ihnen zumindest in einigen Kombinationen eine Korrelation besteht, die im Rahmen der Datenfusion genutzt werden kann, um genauere oder zusätzliche Bodenanalyseergebnisse zu gewinnen. Die Datenfusion kann insbesondere unter auf Basis von Fuzzy-Logik und/oder eines oder mehrerer künstlicher neuronaler Netze implementiert werden.

Bei einigen Ausführungsformen dazu werden die Messdaten von einer Sensorvorrichtung gemäß dem ersten Aspekt der Erfindung, insbesondere gemäß einer oder mehrerer der beschriebenen Ausführungsformen davon, erfasst. Das Verfahren schließt sich dann der eigentlichen In-situ-Messung zur Erfassung der Messdaten an, wobei die Sensorvorrichtung insbesondere, wie oben beschrieben, die Messdaten sowie gegebenenfalls zusätzliche Metadaten dazu mittels seiner Kommunikationsvorrichtung über eine entsprechende Kommunikationsverbindung an eine das Verfahren ausführende zentrale oder räumlich verteilte Vorrichtung übermitteln kann.

Bei einigen Ausführungsformen wird das Verfahren in zumindest einem zentralen Knoten eines Netzwerks, insbesondere einer Cloud-Umgebung oder eines verteilten Rechnernetzwerks, durchgeführt, der konfiguriert ist, zum Empfang der jeweiligen Messdaten mit einer Mehrzahl von Sensorvorrichtungen, insbesondere gemäß dem ersten Aspekt der Erfindung, zur Erfassung der jeweiligen Messdaten in Kommunikationsverbindung zu stehen. Dies erlaubt insbesondere eine leistungsstarke und variable Ressourcennutzung zur Durchführung des Verfahrens. Auch können Änderungen, insbesondere Aktualisierungen einer zur Durchführung des Verfahrens verwendeten Software somit zentral umgesetzt werden, ohne jeweils an die entsprechenden Sensorvorrichtungen verteilt werden zu müssen, sodass sich das Gesamtsystem auf einfache Weise fortentwickeln und aktualisieren lässt.

Ein dritter Aspekt der Erfindung betrifft ein Computerprogramm, das konfiguriert ist, bei seinem Ablauf auf einer Prozessorplattform das Verfahren gemäß dem zweiten Aspekt der Erfindung, insbesondere gemäß einer oder mehrerer der beschriebenen Ausführungsformen davon, auszuführen. Die Prozessorplattform kann einen einzelnen oder eine Mehrzahl von Prozessoren enthalten, und kann lokal zentriert, etwa in einem einzelnen Rechner, oder aber auch über ein dezentrales, verteiltes Rechnernetzwerk hinweg implementiert sein. Insbesondere können die Prozessorplattform und das Computerprogramm dazu auch in der Sensorvorrichtung selbst vorliegen, um diese zur Ausführung des Verfahrens zu ertüchtigen.

Das Computerprogramm kann insbesondere auf einem nichtflüchtigen Datenträger gespeichert sein. Bevorzugt ist dies ein Datenträger in Form eines optischen Datenträgers oder eines Flashspeichermoduls. Dies kann vorteilhaft sein, wenn das Computerprogramm als solches unabhängig von einer Prozessorplattform gehandelt werden soll, auf der das ein bzw. die mehreren Programme auszuführen sind. In einer anderen Implementierung kann das Computerprogramm als eine Datei auf einer Datenverarbeitungseinheit, insbesondere auf einem Server vorliegen, und über eine Datenverbindung, beispielsweise das Internet oder eine dedizierte Datenverbindung, wie etwa ein proprietäres oder lokales Netzwerk, herunterladbar sein. Zudem kann das Computerprogramm eine Mehrzahl von zusammenwirkenden einzelnen Programmodulen aufweisen.

Ein vierter Aspekt der Erfindung betrifft eine Vorrichtung zur Bodenanalyse, wobei die Vorrichtung eingerichtet ist, das Verfahren gemäß dem zweiten Aspekt der Erfindung, insbesondere gemäß einer oder mehrerer der beschriebenen Ausführungsformen davon, auszuführen. Die Vorrichtung kann insbesondere die genannte Prozessorplattform enthalten und demnach insbesondere eine einzelne Datenverarbeitungseinheit, etwa ein Rechner, oder ein dezentrales, verteiltes Rechnernetzwerk aufweisen.

Insbesondere kann die Vorrichtung zur Erfassung der Messdaten bei einigen Ausführungsformen selbst eine Sensorvorrichtung gemäß dem ersten Aspekt der Erfindung, insbesondere gemäß einer oder mehrerer der beschriebenen Ausführungsformen davon, aufweisen. Dies ist insbesondere dann vorteilhaft, wenn die Analyse der Messdaten zur Gewinnung von weiterführenden Bodenanalyseergebnissen in situ, d. h. vor Ort an der Sensorvorrichtung selbst vorgenommen werden soll, was insbesondere auch einen Offlinebetrieb sowie eine von der Qualität einer Kommunikationsverbindung zu einer externen Prozessorplattform unabhängige Bestimmung solcher Ergebnisse erlaubt.

Die in Bezug auf den zweiten Aspekt der Erfindung erläuterten Merkmale und Vorteile gelten entsprechend auch für den dritten und vierten Aspekt der Erfindung.

Weitere Vorteile, Merkmale und Anwendungsmöglichkeiten der vorliegenden Erfindung ergeben sich aus der nachfolgenden detaillierten Beschreibung im Zusammenhang mit den Figuren.

Dabei zeigt
**Fig. 1** schematisch eine Sensorvorrichtung gemäß einer Ausführungsform der Erfindung;
**Fig. 2** schematisch eine modular aufgebaute Sensorvorrichtung gemäß einer weiteren Ausführungsform der Erfindung, bei der neben einem Messmodul auch ein Bedienen/Funkmodul vorgesehen ist;
**Fig. 3A** schematisch eine integrierte Impedanz/Temperatur-Sensorbaugruppe für eine Sensorvorrichtung gemäß einer Ausführungsform der Erfindung und **Fig. 3B** ein vereinfachtes Ersatzschaltbild dazu;
**Fig. 4** schematisch eine Potentialmessbaugruppe, insbesondere eine pH-Sensorbaugruppe, für eine Sensorvorrichtung gemäß einer Ausführungsform der Erfindung;
**Fig. 5** schematisch eine Absorptionsspektrometerbaugruppe für eine Sensorvorrichtung gemäß einer Ausführungsform der Erfindung;
**Fig. 6** eine schematische Übersicht über ein Gesamtsystem zur Bodenanalyse, gemäß einer Ausführungsform der Erfindung; und
**Fig. 7** eine exemplarische Übersicht über verschiedenen Zusammenhänge zwischen einzelnen mittels der Sensoren der Sensorvorrichtung gemäß Fig. 1 bzw. 2erfassbaren Messgrößen, mittels derer im Rahmen einer Datenfusion gemäß dem erfindungsgemäßen Verfahren verschiedene Bodeneigenschaften bestimmt werden können.

In den Figuren werden durchgängig dieselben Bezugszeichen für dieselben oder einander entsprechenden Elemente der Erfindung verwendet.

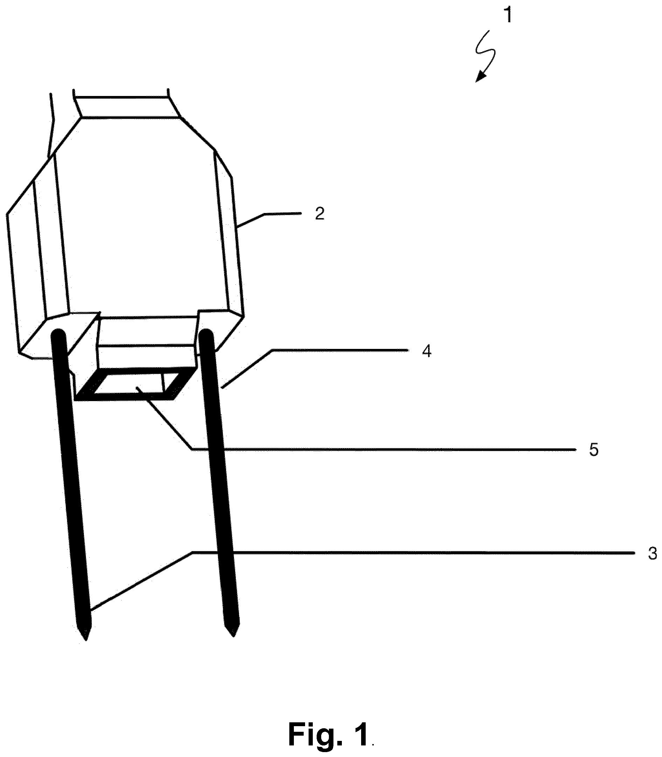

Die in **Fig. 1** gezeigte Sensorvorrichtung 1 gemäß einer Ausführungsform der Erfindung ist als ein Modul ausgebildet, das wiederum mehrere Baugruppen, insbesondere Sensorbaugruppen in einem gemeinsamen Gehäuse 2 aufweist. Eine erste dieser Baugruppen ist eine kombinierte Impedanz/Temperatur-Sensorbaugruppe 3, die zumindest teilweise in einem stab- bzw. dornartigen ersten Trägerelement ausgebildet und zum Einstechen in einen zu analysierenden Boden konfiguriert ist. eine weitere der Baugruppen ist eine Potentialmessbaugruppe 4, insbesondere eine pH-Sensorbaugruppe, die mittels eines zweiten Trägerelements ausgebildet ist, welches wie das erste Trägerelement eine stab- bzw. dornartige Form aufweist und ebenso zum Einstechen in den zu analysierenden Boden ausgebildet ist. Zwischen diesen beiden Baugruppen 3 und 4 sowie in unmittelbarer Nachbarschaft dazu ist als weitere der Baugruppen eine Absorptionsspektrometerbaugruppe 5 angeordnet, die ein Messfenster aufweist, welches so positioniert ist, dass es beim gemeinsamen Einstechen des ersten und des zweiten Trägerelements in den zu analysierenden Boden auf oder oberhalb von diesem zu liegen kommt. Somit sind die drei Sensorbaugruppen auf engem Raum, vorzugsweise auf einer Gesamtfläche von weniger als 100 cm² konzentriert, sodass der Einfluss von Heterogenitäten im zu analysierenden Boden auf die Messergebnisse gering gehalten und insbesondere auf ein Minimum reduziert werden kann. Die Sensorvorrichtung 1 ist als mobile, insbesondere tragbare Einheit ausgebildet hat vorzugsweise ein Gewicht von weniger als 25 kg und eine maximale Erstreckung von weniger als 1 m, vorzugsweise von maximal 0,5 m. Zudem weist die Sensorvorrichtung 1 eine Energieversorgungsvorrichtung auf (nicht dargestellt), die insbesondere in Form eines wiederaufladbaren, elektrochemischen Energiespeichers, wie etwa als Lithium-Ionen-Akkumulator, ausgebildet sein kann.

Die einzelnen Baugruppen, insbesondere Sensorbaugruppen 2, 3 und 4 der Sensorvorrichtung 1 können auch jeweils als individuell entfernbares bzw. austauschbares Modul ausgebildet sein, was es insbesondere ermöglicht, auf einfache Weise und dynamisch verschiedene Sensorkonfigurationen zu erzeugen, sowie die einzelnen Sensorbaugruppen abhängig von deren Alterungs- oder Funktionszustand jeweils einzeln zu warten oder zu ersetzen.

Die Sensorvorrichtung 1 erlaubt es somit pro Messung bis zu vier unterschiedliche Sensorarten einzusetzen und deren verschiedene Messprinzipien auszunutzen, um entsprechende Messdaten zu erhalten, auf deren Basis mithilfe von Korrelation bzw. Datenfusion eine auch über die unmittelbare Messung von Bodeneigenschaften hinausgehende Bestimmung von Bodeneigenschaften mit jedenfalls für viele Anwendungsfälle ausreichend hoher Genauigkeit in-situ erreicht werden kann. Insbesondere können beispielsweise die Impedanz des zu messenden Bodens, die Bodentemperatur, sein Absorptionsspektrum im gesamten UV-VIS-IR-Spektralbereich, sowie sein pH-Wert gleichzeitig und auf dichtestem Raum simultan gemessen werden. Gerade diese eng benachbarte Anordnung der Messgrößen-Aufnehmer der verschiedenen Sensorbaugruppen 2, 3 und 4 erlaubt es erst, eine erfolgreiche Korrelation der Messdaten zum Zwecke der Bestimmung von Bodeneigenschaften mit der für typische, insbesondere agrartechnische, Anwendungen erforderlichen Genauigkeit durchzuführen. Zudem erlaubt es die dichte Anordnung der Messgrößen-Aufnehmer auch, ultra-hochauflösende Bodenkarte zu erstellen, d. h. Bodenkarten mit einem Raster von weniger als 100 cm² Rasterzellenfläche. Die simultane Erfassung der verschiedenen zu messenden Größen ermöglicht außerdem die Darstellung von dynamischen und echten Abhängigkeiten zwischen den einzelnen Messwerten. Insbesondere können so auch Messartefakte bereits in-situ mittels entsprechender Auswertungssoftware, beispielsweise auf Basis von künstlicher Intelligenz, erkannt und entfernt werden, um die Qualität der ursprünglichen Messergebnisse weiter zu steigern.

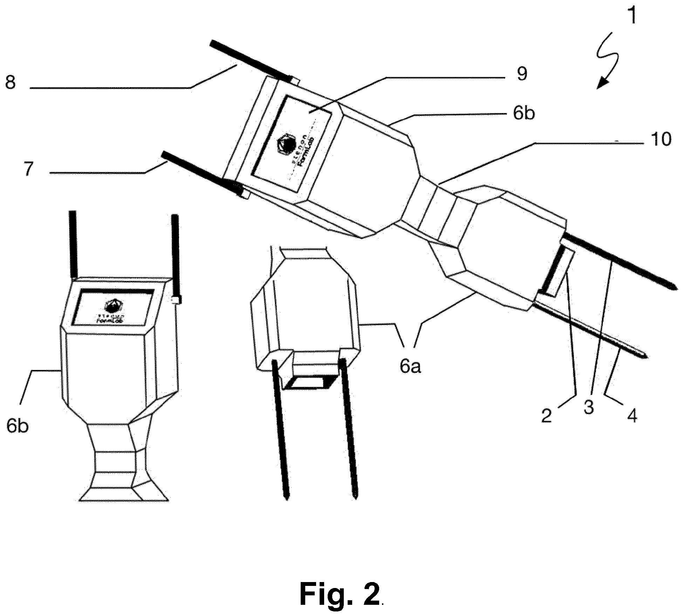

**Fig. 2** zeigt eine modular aufgebaute Sensorvorrichtung 1 gemäß einer weiteren Ausführungsform der Erfindung, die neben einem Sensormodul 6a auch ein damit mittels einer lösbaren Verbindung koppelbares Bedien-/Funkmodul 6b aufweist. Die beiden Module 6a und 6b sind in Fig. 2 einerseits als separate Module (links unten), sowie andererseits im verbundenen Zustand (rechts oben) dargestellt. Die Gehäuse der beiden Module 6a und 6b sind vorzugsweise so ausgeformt, dass sich, wenn die beiden Module miteinander verbunden sind, an der Verbindungsstelle ein für die menschliche Hand bequem greifbarer, insbesondere umfassbarer, Trage- bzw. Handhabungsgriff 10 ausbildet, der insbesondere auch zur Entnahme einer zur Analyse in einen Boden eingestochenen Sensorvorrichtung 1 aus dem Boden eignet.

Der Griff kann insbesondere, wie in Fig. 2 dargestellt, als Verjüngung des Querschnitts der Sensorvorrichtung 1 an der Verbindungsstelle zwischen den beiden Modulen 6a und 6b ausgebildet sein. Das Bedien-/Funkmodul 6b weist eine Positionsbestimmungsvorrichtung 7 auf, mit deren Hilfe, beispielsweise im Zusammenspiel mit einem satellitengestützten Positionserkennungssystem wie etwa GPS, GALILEO oder GLONASS, oder mithilfe von mobilfunkgestützter Positionsbestimmung die Position der Sensorvorrichtung 1, insbesondere während eines Messvorgangs, bestimmt und entsprechende Positionsdaten als zur Messung gehörige Metadaten erzeugt werden können.

Des Weiteren verfügt das Bedien-/Funkmodul 6b über eine Kommunikationsvorrichtung 8, die insbesondere eingerichtet sein kann, mittels Mobilfunktechnologie (z.B. 3G, LTE, 5G), oder einer anderen Funktechnologie, wie etwa LoRa und/oder NB-IoT, eine Datenkommunikation mit einer externen Gegenseite durchzuführen, insbesondere um mittels der Sensorvorrichtung 1 gewonnenen Messdaten zur weiteren Auswertung an ein externes Datenverarbeitungszentrum zu senden, und um gegebenenfalls aus einer solchen Auswertung resultierende Bodenanalyseergebnisse wieder zu empfangen, um sie an der Sensorvorrichtung 1 selbst an einer Mensch-Maschine-Schnittstelle 9 auszugeben. Eine solche Mensch-Maschine-Schnittstelle 9 kann insbesondere in Form einer Anzeigevorrichtung an der Sensorvorrichtung 1 vorgesehen sein, im Hinblick auf eine möglichst raumsparende Lösung vorzugsweise als Bediendisplay, welches sowohl Benutzereingaben, als auch eine Ausgabe von Informationen ermöglicht, wie dies etwa bei einem berührungsempfindlichen Bildschirm der Fall ist.

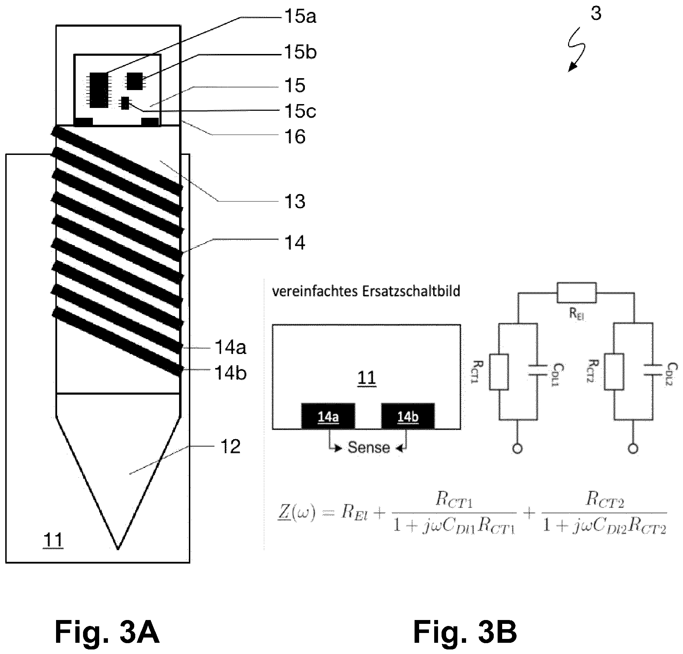

**Fig. 3A** zeigt eine in einen zu analysierenden Boden 11 eingestochene integrierte Impedanz/Temperatur-Sensorbaugruppe 3 für eine Sensorvorrichtung gemäß einer Ausführungsform der Erfindung, die insbesondere in einer Sensorvorrichtung 1 nach Fig. 1 oder Fig. 2 vorgesehen sein kann. **Fig. 3B** zeigt dazu ein vereinfachtes Ersatzschaltbild für den Impedanzmesszweig der Impedanz/Temperatur-Sensorbaugruppe 3.

Die Sensorbaugruppe 3 aus Fig. 3A weist ein erstes Trägerelement 12 in Dornform auf, welches insbesondere aus Metall, bevorzugt aus einem korrosionsbeständigen Metall gefertigt sein kann. Der Dorn kann insbesondere im wesentlichen zylindrische Form haben und an seiner zum Einstechen in den Boden vorgesehenen Stirnseite zur Erleichterung des Einstechens zugespitzt sein. Auf einem Oberflächenbereich, der im eingestochenen Zustand typischerweise mit dem umgebenden Boden in Kontakt kommt, ist eine Passivierungsschicht 13 auf das erste Trägerelement 12 aufgebracht die insbesondere einen oder mehrere Polymerwerkstoffe enthalten kann, und die als elektrischer Isolator wirkt. Auf der Passivierungsschicht 13 sind zwei Leiterbahnen 14 parallel zueinander und ohne sich zu berühren um das erste Trägerelement 12 gewickelt. Die beiden Leiterbahnen 14 sind somit mittels der Passivierungsschicht 13 von dem Trägerelement 12 elektrisch isoliert. An seinem der in den Boden einstechbaren Spitze gegenüberliegenden Stirnseite weist die Impedanz/Temperatur-Sensorbaugruppe 3 ein im Inneren des Trägerelements 12 angeordnetes und mittels einer darüber liegenden Metallkappe 16 (Metallgehäuse) geschützte gedruckte Schaltung (PCB) 15 auf, auf der in Form einer integrierten Schaltung bzw. eines Halbleitersensorbauelements eine Steuerungsvorrichtung 15a,ein Signalvorverstärker 15b sowie ein Temperatursensor 15c vorgesehen sind. Die Metallkappe 16 dient dabei nicht nur den mechanischen Schutz, sondern auch als elektromagnetische Abschirmung des Temperatursensors 15c, der Steuerungsvorrichtung 15a und des Signalvorverstärkers 15b, die in ihrem Inneren gelegen sind. Die Steuerungsvorrichtung 15a dient neben der Steuerung der Sensorbaugruppe 3 zugleich der Impedanzmessung und der Bereitstellung entsprechender Messdaten und ist über den Signalvorverstärker 15b mit den beiden Leiterbahnen 14 jeweils elektrisch verbunden. Auch der Temperatursensor 15c kann mit den Leiterbahnen 14 verbunden sein, wobei sie dann zusätzlich oder alternativ zu dem ersten Trägerelement 12 für den Temperatursensor 15c als Messgrößen-Aufnehmer dienen, während sie in jedem Fall für die Impedanzmessung als Messelektroden dienen.

Die Impedanz/Temperatur-Sensorbaugruppe 3 kann somit, was ihren Impedanzmesszweig betrifft, mittels des in Fig. 3B beschriebenen vereinfachten Ersatzschaltbildes beschrieben werden. Beim Impedanzmessvorgang wird durch die Steuerungsvorrichtung 15a eine definierte Messwechselspannung zwischen eine erste Leiterbahn 14a und die entsprechend zweite Leiterbahn 14b der beiden Leiterbahnen 14 angelegt. Da während des Messvorgangs das erste Trägerelement 11 mit den darauf befindlichen Leiterbahnen 14 in den zu analysierenden Boden 11 eingestochen ist, stehen die beiden Leiterbahnen 14a, 14b mit dem sie dann umgebenden Boden 11 in elektrischen Kontakt, sodass dieser die beiden Leiterbahnen 14a, 14bim Sinne eines elektrischen Widerstands Rₑₗ verbindet. Die beiden Leiterbahnen 14a, 14b weisen im Ersatzschaltbild jeweils selbst einen elektrischen Widerstand R_{CT1} bzw. R_{CT1}, sowie eine parallel dazu geschaltete (parasitäre) Kapazität C_{DL1} bzw. CDL2 auf. Mittels der in Fig. 3B angegebenen Beziehung lässt sich somit in Abhängigkeit von der Frequenz ω der angelegten Messwechselspannung ein Impedanzspektrum Z(ω) bestimmen. Der zur Gewinnung des Impedanzspektrums Z(ω) verwendete Frequenzbereich kann anwendungsspezifisch gewählt werden, und schließt typischerweise den Frequenzbereich von 100 Hz bis 1 MHz ein. Das erste Trägerelement 12 wird während des Impedanz-Messvorgangs idealerweise auf ein Massepotential gelegt und dazu beispielsweise mit dem Null-Pol der Energieversorgung der Sensorvorrichtung 1 elektrisch verbunden, was einer Signalverfälschung von Z(ω) durch externe elektromagnetische Einkopplungen entgegenwirkt.

Auf Basis dieses gewonnenen Impedanzspektrums Z(ω) lässt sich durch weitere Auswertung eine Unterscheidung hinsichtlich der Bodenart, der Bodentextur, der elektrischen Leitfähigkeit, dem Wassergehalt, der lonenkonzentration und der lonenart erreichen, insbesondere mittels die dielektrischer Mischmodelle (zum Beispiel Bruggeman-Modell, Maxwell-Garnett-Modell). Auch quantitative Auswertungen sind auf diese Weise möglich. Simultan mit der Impedanzmessung kann mittels des Temperatursensors zudem eine Temperaturmessung erfolgen, wobei, wie schon erwähnt, die beiden Leiterbahnen 14 und/oder das erste Trägerelement 12 als Messgrößen-Aufnehmer dienen können. Die Impedanz/Temperatur-Sensorbaugruppe 3 kann in einigen Ausführungsformen insbesondere schon die Gesamtheit der Sensoren der Sensorvorrichtung 1 oder gar diese selbst darstellen.

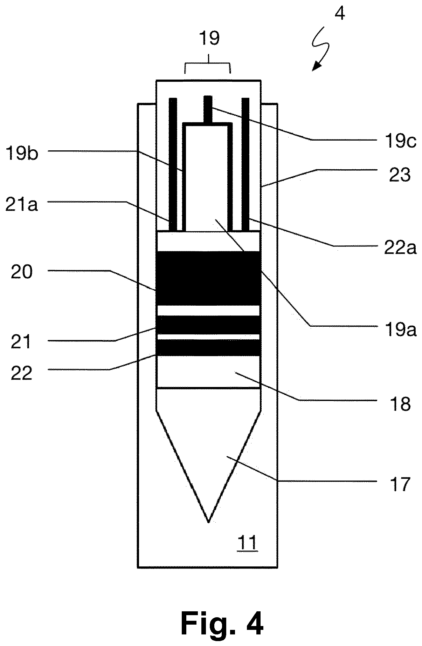

**Fig. 4** zeigt eine in einen zu analysierenden Boden 11 eingestochene Potentialmessbaugruppe 4 gemäß einer Ausführungsform der Erfindung, insbesondere eine pH-Sensorbaugruppe, welche insbesondere in einer Sensorvorrichtung 1 nach Fig. 1 oder Fig. 2 vorgesehen sein kann. Die Potentialmessbaugruppe 4 weist ein zweites Trägerelement 17 in Dornform auf, dessen Form insbesondere im Wesentlichen der des ersten Trägerelements 12 des Impedanz-/Temperatur-Sensorbaugruppe 3 entsprechen kann. Auf einem Oberflächenabschnitt des zweiten Trägerelement 17, der dazu vorgesehen ist, im eingestochenen Zustand mit dem zu analysierenden Boden 11 in Kontakt zu kommen, ist eine Passivierungsschicht 18, insbesondere eine Polymer-Passivierung (z.B. aus HDPE), vorgesehen.

Auf dieser Passivierungsschicht 18 sind in Form ringförmiger Leiterbahnen einerseits eine Metalloxidelektrode 21, sowie eine Kalibrierelektrode 22 angeordnet, mit deren Hilfe bei Kenntnis des elektrischen Widerstands des Bodens 11, der insbesondere mittels der Impedanz/Temperatur-Sensorbaugruppe 3 bestimmt werden kann, ein Zustand, insbesondere ein Schichtdicke, der Metalloxidelektrode 21 mittels Widerstandsmessung bzw. Leitfähigkeitsmessung zwischen den beiden Elektroden 21 und 22, welche durch den Boden 11 elektrisch gekoppelt sind, bestimmt werden kann. Die Schichtdicke kann sodann als Kalibriergröße für die eigentliche Messung eines sauren bzw. basischen Charakters, insbesondere eines pH-Werts, des Bodens 11 dienen. Die Messung kann insbesondere vor jeder pH-Wert-Messung oder zyklisch in vorbestimmten Zeitintervallen erfolgen. Somit ist die Potentialmessbaugruppe in der Lage, eigenständig eine (in-situ) Autokalibrierung vorzunehmen.

Die Metalloxidelektrode 21 sowie die Kalibrierelektrode 22 sind jeweils durch die Passivierungsschicht 18 von dem zweiten Trägerelement 17, welches insbesondere aus Metall gefertigt sein kann, sowie untereinander elektrisch isoliert. Die Kalibrierelektrode 22 kann insbesondere einen leitfähigen Polymerwerkstoff und/oder einen leitfähigen Kompositwerkstoff enthalten oder ganz daraus gefertigt sein. Die Metalloxidelektrode 21 sowie die Kalibrierelektrode 22 weisen jeweils elektrische Anschlüsse 21a bzw. 22a auf, welche insbesondere aus demselben Material wie die zugehörige Elektrode 21 bzw. 22 gefertigt sein können.

Zur Messung des sauren bzw. basischen Charakters des Bodens mittels Potentialmessung weist die Potentialmessbaugruppe 4 des Weiteren eine Elektrolyt/Metall-Referenzelektrode 19 (zum Beispiel AgCl/Ag-Elektrode) auf, welche als in einem als Teil oder Ergänzung des zweiten Trägerelements 17 ausgebildeten Metallgehäuse 23 (Metallkappe) angeordnete Bestandteile ein Elektrolytgefäß 19b zur Aufnahme eines flüssigen oder pastösen Elektrolyten 19a als Elektrolyt-Referenzelektrode, sowie eine Metall-Referenzelektrode 19c, welche mit den Elektrolytgefäß 19b und dem darin befindlichen Elektrolyt 19a in elektrisch leitender Verbindung steht, enthält. Mittels des Metallgehäuses 23 wird insbesondere ein robuster mechanischer Schutz der Referenzelektrode 19 erreicht.

Die Kombination der Metalloxidelektrode 21, der Elektrolyt/Metall-Referenzelektrode 19, sowie eines dazwischen an der Oberfläche des zweiten Trägerelements 17 angeordneten lonendiaphragmas 20, welches mit der Elektrolyt/Metall-Referenzelektrode 19 in ionenleitender Verbindung steht, und welches über den umgebenden Boden 11 während des Messvorgangs ebenfalls in eine ionenleitende Verbindung mit der Metalloxidelektrode gebracht werden kann, stellt eine Messvorrichtung zur Messung des sauren oder basischen Charakters des Bodens 11 auf Basis der bereits oben genannten chemischen Redox-Reaktion:

xMe + yH₂O ↔ MeₓO_{y} + y2H⁺+ y2e⁻

dar, deren Reaktionsgleichgewicht maßgeblich durch die im Boden 11 vorhandene Konzentration von Wasserstoff-Ionen (H⁺) mitbestimmt ist, so dass sich mittels der bei der Messung auftretenden lonenströme bzw. der auftretenden Potentialdifferenz zwischen der Metalloxidelektrode 21 und der Elektrolyt/Metall-Referenzelektrode 19 unter Berücksichtigung einer Kalibrierung auf Basis der beschriebenen Messung des Zustands der Metalloxidelektrode 21 die H⁺-Ionenkonzentration im Boden und somit dessen pH-Wert bestimmen lässt.

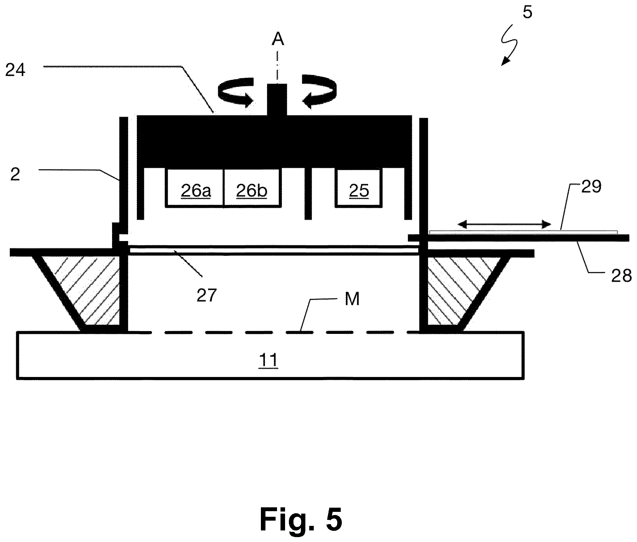

**Fig. 5** zeigt schematisch eine Absorptionsspektrometerbaugruppe 5 für eine erfindungsgemäße Sensorvorrichtung, die insbesondere die Sensorvorrichtung 1 gemäß Fig. 1 oder Fig. 2 sein kann. Dementsprechend wird nachfolgend wieder auf die Sensorvorrichtung 1 Bezug genommen. Die Absorptionsspektrometerbaugruppe 5 weist einen in das Gehäuse 2 der Sensorvorrichtung 1 zwischen die beiden Sensorbaugruppen 3 und 4 eingepassten, um eine Achse A rotierbaren und im Wesentlichen scheibenförmigen Träger 24 auf, dessen eine Scheibenfläche einer Öffnung des Gehäuses 2 zugewandt ist, die als Messöffnung bzw. Messfenster der Absorptionsspektrometerbaugruppe 5 dient. Die an ihrer äußeren geometrischen Grenze liegende virtuelle Fläche dieser Öffnung kann auch als Messfläche M bezeichnet werden, die im Messbetrieb typischerweise, zumindest im Wesentlichen, parallel zur Bodenoberfläche des zu analysierenden Bodens 11 zu liegen kommt, oder mit dieser zusammenfällt und Fig. 5 als gestrichelte Linie dargestellt ist. Der Träger 24 ist gegenüber dieser Messfläche M so positioniert, dass er im Messbetrieb oberhalb der Bodenoberfläche zu liegen kommt, wobei ein Mindestabstand durch die Formgebung des Gehäuses 2 definiert ist. An der der Messfläche zugewandten Seite des Trägers 24 sind auf diesem zwei (oder mehrere) einzelne MEMS-Absorptionsspektrometer 26a, 26b angeordnet, die jeweils zumindest teilweise verschiedene Spektralbereiche abdecken, und kumulativ einen Spektralbereich UV-VIS-NIR abdecken, der insbesondere den Spektralbereich von 350 nm bis 1700 nm einschließt. Der Einsatz von MEMS-Technologie zur Erzeugung der Absorptionsspektrometer ermöglicht dabei die Herstellung besonders kleiner und somit raumeffizienter Bauformen.

Zudem ist auf derselben Seite des Trägers 24 eine Quelle 25 für elektromagnetische Strahlung vorgesehen, beispielsweise eine Halogenlampe, deren Strahlung diesem Spektralbereich UV-VIS-NIR abdeckt. Dabei sind die Quelle 25 und die Absorptionsspektrometer 26a, 26b relativ zueinander so angeordnet, bzw. durch eine auf dem Träger 24 ausgebildete Blende so voneinander optisch getrennt, dass die Strahlung der Quelle 25 nur auf indirektem Wege in Form reflektierter Strahlung zu den Absorptionsspektrometer 26a, 26b gelangen kann.

Zusätzlich weist die Absorptionsspektrometerbaugruppe 5 eine Schutzoptik 27 auf, die insbesondere in Form einer aus kratzfestem und im genannten Spektralbereich, zumindest weitgehend, transparenten Material bestehenden Scheibe, beispielsweise einer Saphirglasscheibe, mit einer hydrophilen und Kratzschutz verbessernden Nanobeschichtung ausgebildet sein kann. Die Nanobeschichtung erleichtert dabei das Reinhalten der Optik sowie deren Reinigung und erhöht ihre mechanische Robustheit. Die Schutzoptik 27 ist dabei zwischen dem Träger 24 mit den darauf befindlichen optischen Komponenten 25, 26a, 26b sowie der Messfläche (in einem Abstand, z.B. ca. 3cm, davon) angeordnet, die optischen Komponenten gegen schädliche Einwirkungen von außen, insbesondere durch den zu analysierenden Boden 11, schützen kann, etwa gegen Staub und Feuchtigkeit sowie mechanische bewirkte Beschädigungen.

Des Weiteren weist die Absorptionsspektrometerbaugruppe 5 eine Schließ- bzw. Shuttervorrichtung 28 auf, wobei es sich im Wesentlichen um eine, bevorzugt parallel zu der Schutzoptik 27, in den zwischen dem Träger 24 mit den optischen Komponenten 25, 26a, 26b und der Schutzoptik 27 definierten Raumbereich einfahrbare (und wieder ausfahrbare) scheibenförmige Blende handelt. Auf ihrer den optischen Komponenten 25, 26a, 26b zugewandten Seite ist diese Blende mit einer Kalibrierbeschichtung 29, beispielsweise Spectralon, beschichtet. Spectralon ist ein Material aus gesintertem PTFE, das einen äußerst hohen und gleichmäßigen Reflexionsgrad sowohl im Ultravioletten (UV) als auch im Sichtbaren (VIS) und im nahen Infrarotbereich (NIR) des elektromagnetischen Spektrums aufweist. Es zeigt lambertsches Reflexionsverhalten, reflektiert also sehr diffus bzw. matt. Die Kalibrierbeschichtung 29 dient als Kalibrierungsreferenz, mit deren Hilfe die Absorptionsspektrometer 26a, 26b in situ kalibriert werden können, wenn die Blende dazu in den Raumbereich zwischen den Absorptionsspektrometern 26a, 26b und der Schutzoptik 27 eingefahren ist. Während des Messvorgangs zur Bodenanalyse ist die Blende dagegen ausgefahren, um den Strahlengang zwischen den optischen Komponenten 25, 26a, 26b und dem Boden 11 nicht zu stören.

Die Absorptionsspektrometerbaugruppe 5 ist zudem so konfiguriert, dass im Messbetrieb, wenn die Bodenoberfläche des zu analysierenden Bodens 11, zumindest im Wesentlichen, mit der Messfläche zusammenfällt, der Träger 24 um die dann im Wesentlichen senkrecht zur Messfläche stehende Drehachse A rotiert wird, während die Quelle 25 und die beiden Absorptionsspektrometer 26a, 26b aktiv sind, um auf Basis der an der Bodenoberfläche reflektierten Strahlung der Quelle 25 an den Absorptionsspektrometer 26a, 26b ein Absorptionsspektrum im genannten Spektralbereich aufzunehmen.

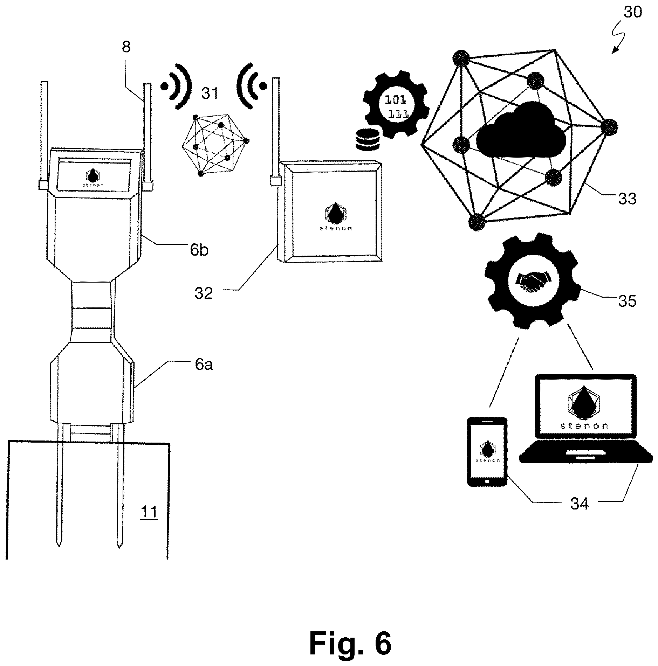

**Fig. 6** zeigt eine schematische Übersicht über ein (Gesamt-)system 30 zur Bodenanalyse, gemäß einer Ausführungsform der Erfindung. Das System 30 weist eine oder typischerweise mehrere Sensorvorrichtungen, insbesondere Sensorvorrichtungen 1 gemäß den Figuren 1 oder 2 auf (von denen hier nur eine gezeigt ist) die vor Ort, d. h. in-situ, zur Gewinnung von Eigenschaften eines zu analysierenden Bodens 11 charakterisieren Messdaten dienen. Diese Messdaten können sodann von der jeweiligen Sensorvorrichtung 1 mittels der Kommunikationsvorrichtung 8 über eine Kommunikationsverbindung, die insbesondere als Blockchain-Transfer ausgestaltet sein kann, an eine geräteexterne Gegenseite 33 übermittelt werden, die insbesondere in Form eines oder mehrerer Netzwerkknoten (zum Beispiel Server) in einem Rechennetzwerk bzw. einer Cloud-Umgebung realisiert sein können.

Im dargestellten Beispiel erfolgt die Übermittlung mehrstufig, indem die Messdaten sowie gegebenenfalls zugehörige Metadaten zur Messung zunächst über eine drahtlose Kommunikationsverbindung, welche insbesondere mittels der LoRa- oder der NB-IoT- Funktechnologien realisiert sein kann, an einen Gateway 32 übermittelt werden, der beispielsweise am Hof eines Landwirts, der das System 30 nutzt, positioniert sein kann. Von diesem Gateway 32 können die Messdaten und Metadaten zur Auswertung weiter an die Gegenseite 33 übermittelt werden, beispielsweise auf klassischem Wege über eine drahtlose oder drahtgebundene Internetverbindung. Auch ist wiederum ein bevorzugt ein Blockchain-Transfer vorgesehen, so dass die gesamte Kommunikation zwischen der Sensorvorrichtung 1 und der Gegenseite 33 mittels Blockchain-Technologie umgesetzt wird. Dieser Kommunikationsweg ist bidirektional, sodass er auch in umgekehrter Richtung nutzbar ist, insbesondere zur Übermittlung von durch die Gegenseite 33 auf Basis der an sie übermittelten Messdaten und Metadaten gewonnenen Analysedaten an die jeweilige Sensorvorrichtung 1. Die von der jeweiligen Sensorvorrichtung 1 erfassten Metadaten können je nach Ausgestaltung insbesondere Informationen zum Zeitpunkt und dem Ort einer vorgenommenen Bodenmessung, sowie eine eindeutige Geräteidentifikation und/oder Benutzeridentifikation enthalten.

Zusätzlich oder alternativ kann eine weitere Kommunikationsverbindung 35 zwischen der Gegenseite 33 und einem oder mehreren Benutzerendgeräten 34 vorgesehen sein, die insbesondere als Fernzugriff (Remote-Access), etwa über ein Webportal, ausgestaltet und wiederum vorteilhaft mittels Blockchain-Technologie umgesetzt sein kann. Sämtliche Kommunikationsverbindungen im System sind bevorzugt zum Zwecke der Erhaltung der Datensicherheit und zum Schutz vor Manipulationen verschlüsselt, beispielsweise mittels bekannter asymmetrischer oder symmetrischer Verschlüsselungsverfahren. Über die Kommunikationsverbindung 35 besteht ein weiterer Weg, auf dem die gewonnenen Analysedaten zugreifbar sind. Beispielsweise kann der Landwirt oder Gartenbauer auf diese Weise auch in größeren zeitlichen Abstand von der Durchführung der Messung, etwa von seinem Hof aus oder auch von unterwegs, über ein entsprechendes Endgerät 34 auf die Analysedaten zugreifen, ohne dass er dazu die Sensorvorrichtung 1 bei sich haben muss.

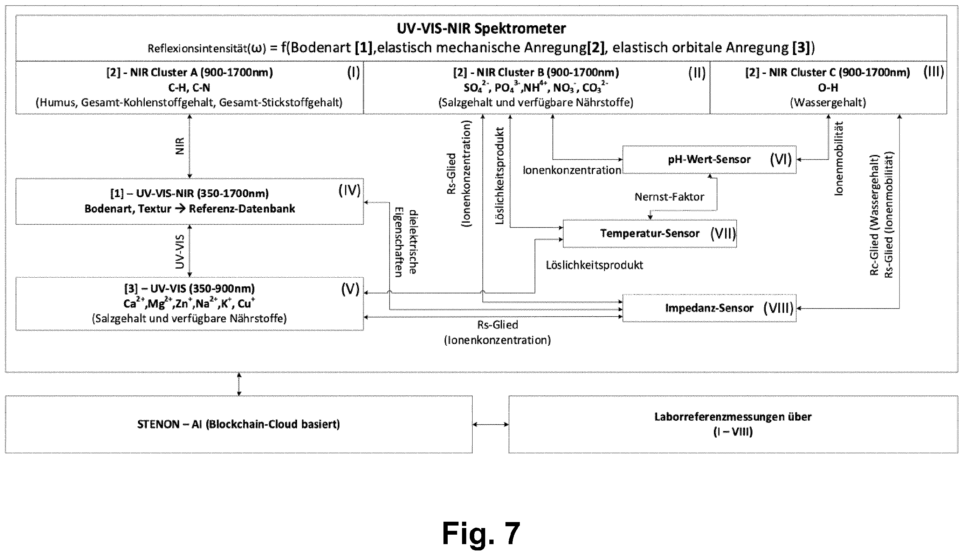

**Fig.** 7 stellt eine exemplarische Übersicht über verschiedenen Zusammenhänge zwischen einzelnen mittels der Sensoren der Sensorvorrichtung gemäß Fig. 1 bzw. 2erfassbaren Messgrößen dar, mittels derer im Rahmen einer Datenfusion (oder hier gleichbedeutend Sensorfusion) gemäß dem erfindungsgemäßen Verfahren verschiedene Bodeneigenschaften bestimmt werden können. Dabei sind die Zusammenhänge mittels entsprechender beschrifteter Pfeile gekennzeichnet, wobei die Beschriftungen diejenigen physikalischen bzw. chemischen Größen angeben, die im Rahmen der Datenfusion insbesondere genutzt werden können, um Verknüpfungen zwischen den verschiedenen unmittelbaren von den Sensorbaugruppen 3 bis 5 gelieferten Messgrößen zu bilden, die es ermöglichen zusätzliche abgeleitete Bodeneigenschaften zu bestimmen und/oder die Genauigkeit der erzielbaren Ergebnisse zu erhöhen. Insbesondere können auf diese Weise eine Reihe wichtiger Parameter für die Agrar- und Gartenbauwirtschaft bestimmt werden, wozu insbesondere der Gesamt-Stickstoffgehalt, der Gesamthumusgehalt, das Verhältnis von Stickstoff zu organischem Material, das verfügbare Phosphat, das verfügbare Kalium, des verfügbare Magnesium, die elektrische Leitfähigkeit, Bodenfeuchte und der pH-Wert des Bodens gehören.

Während vorausgehend wenigstens eine beispielhafte Ausführungsform beschrieben wurde, ist zu bemerken, dass eine große Anzahl von Variationen dazu existiert. Es ist dabei auch zu beachten, dass die beschriebenen beispielhaften Ausführungsformen nur nichtlimitierende Beispiele darstellen, und es nicht beabsichtigt ist, dadurch den Umfang, die Anwendbarkeit oder die Konfiguration der hier beschriebenen Vorrichtungen und Verfahren zu beschränken. Vielmehr wird die vorausgehende Beschreibung dem Fachmann eine Anleitung zur Implementierung mindestens einer beispielhaften Ausführungsform liefern, wobei sich versteht, dass verschiedene Änderungen in der Funktionsweise und der Anordnung der in einer beispielhaften Ausführungsform beschriebenen Elemente vorgenommen werden können, ohne dass dabei von dem in den angehängten Ansprüchen jeweils festgelegten Gegenstand sowie seinen rechtlichen Äquivalenten abgewichen wird.

### BEZUGSZEICHENLISTE

- 1: Sensorvorrichtung
- 2: Gehäuse
- 3: Impedanz/Temperatur-Sensorbaugruppe
- 4: Potentialmessbaugruppe, insbesondere pH-Sensorbaugruppe
- 5: Absorptionsspektrometerbaugruppe
- 6a: Sensormodul
- 6b: Bedien-/Funkmodul
- 7: Positionsbestimmungsvorrichtung
- 8: Kommunikationsvorrichtung
- 9: Mensch-Maschine-Schnittstelle, insbesondere Bediendisplay
- 10: Trage- bzw. Handhabungsgriff
- 11: Boden
- 12: (erstes) Trägerelement, in Dornform
- 13: Passivierung, insbesondere Polymer-Passivierung, des ersten Trägerelements
- 14: Leiterbahnen
- 14a: erste Leiterbahn
- 14b: zweite Leiterbahn
- 15: integriertes PCB mit Steuerungsvorrichtung und Temperatursensor
- 15a: Steuerungsvorrichtung
- 15b: Signalvorverstärker
- 15c: Temperatursensor
- 16: Metallgehäuse, insbesondere Metallkappe, des ersten Trägerelements
- 17: (zweites) Trägerelement, in Dornform
- 18: Passivierung, insbesondere Polymer-Passivierung, des zweiten Trägerelements
- 19: Elektrolyt/Metall-Referenzelektrode
- 19a: Elektrolyt-Referenzelektrode (Elektrolyt)
- 19b: Elektrolytgefäß
- 19c: Metall-Referenzelektrode
- 20: lonendiaphragma
- 21: Metalloxidelektrode
- 21a: Anschluss der Metalloxidelektrode
- 22: Kalibrierelektrode
- 22a: Anschluss der Kalibrierelektrode
- 23: Metallgehäuse, insbesondere Metallkappe, des zweiten Trägerelements
- 24: rotierbarer Träger mit Drehachse A
- 25: elektromagnetische Strahlungsquelle
- 26a,b: MEMS-Absorptionsspektrometer mit Messfläche M
- 27: (Schutz-)Optik, insbesondere Saphirglas mit hydrophiler Nanobeschichtung
- 28: Shuttervorrichtung
- 29: Kalibrierreferenz, insbesondere Kalibrierbeschichtung
- 30: System zur In-situ-Bodenanalyse
- 31: Kommunikationsverbindung, insbesondere Blockchain-Transfer
- 32: Gateway
- 33: Gegenseite, insbesondere Blockchain/Cloud-Umgebung oder lokale Auswertevorrichtung
- 34: Benutzerendgerät
- 35: Fernzugriff

## Patentansprüche

1. Sensorvorrichtung (1) zur in-situ Bodenanalyse, aufweisend:
eine Sensorbaugruppe mit einem oder mehreren Sensoren, die einzeln oder kumulativ zur simultanen in-situ Messung der folgenden Bodeneigenschaften eines zu analysierenden Bodens (11) und zur Bereitstellung entsprechender jeweiliger Messdaten konfiguriert sind:
(a) Impedanzspektrum;
(b) Temperatur; und
(c) Absorptionsspektrum in einem von NIR bis UV reichenden Spektralbereich NIR-VIS-UV;
wobei:
der bezüglich ihrer jeweiligen Messgrößen-Aufnehmer definierte Abstand zwischen je zwei der Sensoren der Sensorbaugruppe einen Wert von 10 cm nicht überschreitet;
die Sensor-Baugruppe konfiguriert ist, zur in-situ Messung des Impedanzspektrums (Z(ω)) einen Wechselstromwiderstand eines zu vermessenden Bodenabschnitts in Abhängigkeit von der Frequenz (ω) einer an den Bodenabschnitt angelegten Messwechselspannung zu messen; und
die Sensor-Baugruppe konfiguriert ist, zur in-situ Messung des Absorptionsspektrums eine an der Bodenoberfläche des zu analysierenden Bodens (1) reflektierte Strahlung im genannten Spektralbereich aufzunehmen.

2. Sensorvorrichtung (1) gemäß Anspruch 1, wobei die Sensorbaugruppe ferner einen oder mehrere Sensoren aufweist, die einzeln oder kumulativ dazu konfiguriert sind, eine mit den anderen Messungen simultane in-situ Messung eines sauren oder basischen Charakters des zu analysierenden Bodens (11) durchzuführen und entsprechende Messdaten bereitzustellen.

3. Sensorvorrichtung (1) gemäß Anspruch 1 oder 2, wobei die Sensorbaugruppe zur In-situ-Erfassung eines Impedanzspektrums des zu analysierenden Bodens (11) einen Impedanzsensor enthält, der aufweist:
ein erstes Trägerelement (12);
zwei auf dem ersten Trägerelement (12) aber von diesem und untereinander elektrisch isoliert angeordnete Leiterbahnen (14), von denen zumindest eine einen elektrisch leitfähigen korrosionsbeständigen Polymer- oder Kompositwerkstoff enthält;
eine Steuerungsvorrichtung (15a), die konfiguriert ist, zwischen den beiden Leiterbahnen (14) eine Wechselspannung anzulegen, deren Frequenz über einen vorbestimmten Frequenzbereich hinweg zu variieren, und dabei im Betrieb der Sensorvorrichtung (1), wenn diese so in den zu analysierenden Boden (11) eingebracht ist, dass die Leiterbahnen (14) mit diesem in elektrischen Kontakt stehen, ein Impedanzspektrum des zu analysierenden Bodens (11) in Reaktion auf die an ihn über die Leiterbahnen (14) angelegte Wechselspannung zu erfassen und in Form entsprechender Messdaten bereitzustellen.

4. Sensorvorrichtung (1) gemäß Anspruch 3, wobei das erste Trägerelement zumindest in einem von den Leiterbahnen (14) überdeckten Bereich elektrisch leitfähig ist und die Steuerungsvorrichtung (15a) des Weiteren konfiguriert ist, während der Erfassung des Impedanzspektrums des zu analysierenden Bodens (11) das elektrische Potential dieses zumindest einen Bereichs auf ein Massepotential zu legen.

5. Sensorvorrichtung (1) gemäß Anspruch 3 oder 4, wobei der vorbestimmte Frequenzbereich den Bereich von 100 Hz bis 1 MHz einschließt.

6. Sensorvorrichtung (1) gemäß einem der Ansprüche 3 bis 5, wobei:
das erste Trägerelement als zumindest abschnittsweise hohler Dorn zum zumindest abschnittsweisen Einbringen in den zu analysierenden Boden (11) ausgebildet ist,
wobei auf der Oberfläche des Dorns eine Isolationsschicht aufgebracht ist, und auf der wiederum die beiden Leiterbahnen (14) angeordnet sind; und die Steuerungsvorrichtung (15a) im Inneren eines hohlen Abschnitts des ersten Trägerelements (12) angeordnet ist.

7. Sensorvorrichtung (1) gemäß einem der Ansprüche 3 bis 6, wobei die Sensorbaugruppe zur Erfassung einer Temperatur des zu analysierenden Bodens (11) einen Temperatursensor (15c) enthält, wobei dieser zusammen mit dem Impedanzsensor als integrierte Impedanz/Temperatur-Sensorbaugruppe (3) ausgebildet ist, die konfiguriert ist, simultan und in-situ sowohl ein Impedanzspektrum als auch eine Temperatur des zu analysierenden Bodens (11) zu erfassen und jeweils in Form entsprechender Messdaten bereitzustellen.

8. Sensorvorrichtung (1) gemäß einem der vorausgehenden Ansprüche, wobei die Sensorbaugruppe zur In-situ-Erfassung eines Absorptionsspektrums des zu analysierenden Bodens (11) eine Absorptionsspektrometerbaugruppe (5) enthält, die aufweist:
eine Quelle (25) für elektromagnetische Strahlung, deren Strahlung den genannten von NIR bis UV reichenden Spektralbereich NIR-VIS-UV abdeckt;
zumindest zwei MEMS-Absorptionsspektrometer (26a, 26b), deren spektrale Abdeckung sich zumindest für Teilbereiche des elektromagnetischen Spektrums unterscheidet, so dass kumulativ durch die Gesamtheit der MEMS-Absorptionsspektrometer (26a, 26b) ein Absorptionsspektrum des zu analysierenden Bodens (11) erfassbar ist, welches sowohl Anteile im NIR-Bereich als auch im VIS-Bereich als auch im UV Bereich aufweist.

9. Sensorvorrichtung (1) nach Anspruch 8, wobei die Absorptionsspektrometerbaugruppe (5) des Weiteren einen beweglichen Träger (4) aufweist, auf dem die Absorptionsspektrometer so angeordnet sind, dass sie bei einer Bewegung des Trägers (24) relativ zu einer virtuellen Messfläche, an der im Messbetrieb der Sensorvorrichtung (1) der zu analysierende Boden (11) zu liegen kommt, einen dabei von den Absorptionsspektrometern abzutastenden Bereich des Bodens (11) spektrometrisch vermessen können, um ein über den abzutastenden Bereich integriertes Absorptionsspektrum zu erfassen.

10. Sensorvorrichtung (1) nach Anspruch 8 oder 9, wobei die Absorptionsspektrometerbaugruppe (5) des Weiteren eine bewegliche Shuttervorrichtung (28) aufweist, die konfiguriert ist, temporär eine Blende in einen zwischen den Absorptionsspektrometern und der Messfläche definierten Raumbereich einzufahren, wobei auf der den Absorptionsspektrometern zugewandten Seite der Blende eine Kalibrierreferenz (29) zur Kalibrierung zumindest eines der Absorptionsspektrometer angeordnet ist.

11. Sensorvorrichtung (1) nach einem der Ansprüche 8 bis 10, wobei die Absorptionsspektrometerbaugruppe (5) des Weiteren eine in einem dem aufzunehmenden Absorptionsspektrum entsprechenden Wellenlängenbereich, zumindest im Wesentlichen, optisch transparente Optik (27) aufweist, die in dem Raumbereich zwischen den Absorptionsspektrometern und der Messfläche angeordnet ist, um diese räumlich voneinander abzutrennen; wobei die Optik (27) auf ihrer der Messfläche zugewandten Seite mit einer hydrophilen und Kratzschutz verbessernden Nanobeschichtung versehen ist.

12. Sensorvorrichtung (1) gemäß einem der vorausgehenden Ansprüche, wobei die Sensorbaugruppe zur in-situ-Erfassung eines sauren oder basischen Charakters des zu analysierenden Bodens (11) eine Potentialmessbaugruppe (4) enthält, die aufweist:
ein zweites Trägerelement (17);
eine in oder an dem zweiten Trägerelement angeordnete Elektrolyt/Metall-Referenzelektrode (19);
eine an einer beim Messbetrieb zur Kontaktierung des zu analysierenden Bodens (11) vorgesehenen Oberfläche des zweiten Trägerelements (17) angeordnete Metalloxidelektrode (21);
ein am zweiten Trägerelement zwischen der Metalloxidelektrode (21) und der Elektrolyt/Metall-Referenzelektrode (19) angeordnetes und mit der Elektrolyt/Metall-Referenzelektrode (19) in Kontakt stehendes lonendiaphragma (20);
eine an der zur Kontaktierung des zu analysierenden Bodens (11) vorgesehenen Oberfläche des zweiten Trägerelements (17) angeordnete und von der Metalloxidelektrode (21) elektrisch isolierte korrosionsbeständige Kalibrierelektrode (22); und
eine Messeinrichtung, die konfiguriert ist:
zur Bestimmung eines aktuellen Zustands der Metalloxidelektrode (21) einen zwischen der Kalibrierelektrode (22) und der Metalloxidelektrode (21) auftretenden elektrischen Widerstand und/oder eine dazwischen auftretende elektrische Kapazität zu messen, wenn diese beiden Elektroden jeweils mit dem zu analysierenden Boden (11) in Kontakt stehen; und
zur Bestimmung eines sauren oder basischen Charakters des zu analysierenden Bodens (11) eine zwischen der Referenzelektrode und der Metalloxidelektrode (21) auftretende elektrische Potentialdifferenz unter Berücksichtigung einer zuvor auf Basis des bestimmten aktuellen Zustands der Metalloxidelektrode (21) festgelegten Messkalibrierung zu messen, wenn diese beiden Elektroden jeweils mit dem zu analysierenden Boden (11) in Kontakt stehen.

13. Sensorvorrichtung (1) gemäß Anspruch 12, wobei die Kalibrierelektrode (22) aus einem Material gefertigt ist, das einen elektrisch leitfähigen und korrosionsbeständigen Polymer- oder Kompositwerkstoff enthält.

14. Sensorvorrichtung (1) gemäß Anspruch 12 oder 13 wobei das zweite Trägerelement als Dorn zum zumindest abschnittsweisen Einbringen in den zu analysierenden Boden (11) ausgebildet ist, wobei auf der Oberfläche des Dorns eine Isolationsschicht aufgebracht ist, auf der die Metalloxidelektrode (21), das lonendiaphragma (20), und/oder die Kalibrierelektrode (22) angeordnet sind.

15. Sensorvorrichtung (1) gemäß einem der vorausgehenden Ansprüche, des Weiteren aufweisend eine Kommunikationsvorrichtung (8) zur Übermittlung von erfassten Messdaten zu deren Auswertung an eine bezüglich der Sensorvorrichtung (1) externe Gegenseite.

16. Sensorvorrichtung (1) gemäß Anspruch 15, wobei die Kommunikationsvorrichtung (8) konfiguriert ist, die Messdaten drahtlos mittels einer Kommunikation auf Basis der LoRa-Funktechnologie und/oder der NarrowBand-Internet of Things, NB-loT,-Funktechnologie zu übermitteln.

17. Sensorvorrichtung (1) gemäß Anspruch 15 oder 16, des Weiteren aufweisend eine sichere Speichervorrichtung zur gegen unautorisierten Zugriff geschützten Ablage einer eindeutigen Geräteidentifikation der Sensorvorrichtung (1) und/oder zumindest eines kryptographischen Schlüssels zur Verschlüsselung von mittels der Kommunikationsvorrichtung (8) übermittelten Mess- und/oder Metadaten.

18. Sensorvorrichtung (1) gemäß einem der Ansprüche 15 bis 17, wobei die Kommunikationsvorrichtung (8) des Weiteren konfiguriert ist, zu übermittelnde Mess- und/oder Metadaten in eine als externe Gegenseite auftretende Blockchain zu schreiben oder eine andere externe Gegenseite dazu zu veranlassen, die an sie übermittelten Mess- und/oder Metadaten in eine Blockchain zu schreiben.

19. Sensorvorrichtung (1) gemäß Anspruch 18, wobei die Sensorvorrichtung (1) konfiguriert ist, eine Authentifizierung eines Nutzers der Sensorvorrichtung (1) vorzunehmen und ein Übermitteln der Mess- und/oder Metadaten an eine externe Gegenseite nur dann zuzulassen, wenn die Authentifizierung erfolgreich verlaufen ist.

20. Sensorvorrichtung (1) gemäß einem der vorausgehenden Ansprüche, des Weiteren aufweisend eine Positionsbestimmungsvorrichtung (7) zur Bestimmung einer aktuellen Position der Sensorvorrichtung (1) und zur Bereitstellung entsprechender die Position kennzeichnender Metadaten.

21. Computer-implementiertes Verfahren zur Bodenanalyse, aufweisend:
Empfangen von Messdaten bezüglich der folgenden Bodeneigenschaften eines zu analysierenden Bodens (11), wobei die Messdaten folgendes repräsentieren:
(a) ein Impedanzspektrum (Z(ω)), das den Wechselstromwiderstand eines zu vermessenden Bodenabschnitts in Abhängigkeit von der Frequenz (ω) einer an den Bodenabschnitt, angelegten Messwechselspannung angibt,
(b) eine Temperatur,
(c) ein Absorptionsspektrum in einem von NIR bis UV reichenden Spektralbereich NIR-VIS-UV; und
Bestimmen von zumindest einer der Bodeneigenschaften oder zumindest einer daraus abgeleiteten Bodeneigenschaft auf Basis einer Verknüpfung der empfangenen Messdaten mittels Datenfusion zur Gewinnung eines jeweiligen Messergebnisses für die zumindest eine zu bestimmende Bodeneigenschaft.

22. Computer-implementiertes Verfahren gemäß Anspruch 21, des Weiteren aufweisend:
Empfangen von Messdaten bezüglich der folgenden Bodeneigenschaft des zu analysierenden Bodens (11): saurer oder basischer Charakter.

23. Computer-implementiertes Verfahren gemäß Anspruch 21 oder 22, wobei die Messdaten von einer Sensorvorrichtung (1) gemäß einem der Ansprüche 1 bis 20 erfasst werden.

24. Computer-implementiertes Verfahren gemäß einem der Ansprüche 21 bis 23, wobei das Verfahren in zumindest einem zentralen Knoten (33) eines Netzwerks durchgeführt wird, der konfiguriert ist, zum Empfang der jeweiligen Messdaten mit einer Mehrzahl von Sensorvorrichtungen (1) zur Erfassung der jeweiligen Messdaten in Kommunikationsverbindung (31) zu stehen.

25. Computerprogramm, das konfiguriert ist, bei seinem Ablauf auf einer Prozessorplattform das computer-implementierte Verfahren gemäß einem der Ansprüche 21 bis 24 auszuführen.

26. Vorrichtung (33) zur Bodenanalyse, wobei die Vorrichtung eingerichtet ist, das computer-implementierte Verfahren gemäß einem der Ansprüche 21 bis 24 auszuführen.

27. Vorrichtung (1, 33) gemäß Anspruch 26, aufweisend eine Sensorvorrichtung (1) gemäß einem der Ansprüche 1 bis 20 zur Erfassung der Messdaten.

## Claims

1. A sensor device (1) for in situ soil analysis, comprising:
a sensor assembly with one or more sensors which are configured individually or cumulatively for simultaneous in situ measurement of the following soil properties of a soil (11) to be analyzed and for providing corresponding respective measurement data:
(a) impedance spectrum;
(b) temperature; and
(c) absorption spectrum in a spectral range which extends from NIR to UV, NIR-VIS-UV
wherein:
the distance between each two sensors of the sensor assembly, defined in relation to their respective measurand transducers, does not exceed a value of 10 cm;
in order to measure, in situ, the impedance spectrum (Z(ω)), the sensor assembly is configured to measure an alternating current resistance of a portion of soil to be measured, as a function of the frequency (ω) of an alternating measuring voltage which is applied to the portion of soil; and
in order to detect, in situ, the absorption spectrum, the sensor assembly is configured to measure within said spectral range radiation reflected at the soil surface of the soil (11) to be analyzed.

2. The sensor device (1) according to claim 1, wherein the sensor assembly further comprises one or more sensors which are configured individually or cumulatively for measuring, in situ and simultaneously with the other measurements, an acidic or basic character of the soil (11) to be analyzed and for providing corresponding measurement data.

3. The sensor device (1) according to claim 1, wherein the sensor assembly includes an impedance sensor for in situ detection of an impedance spectrum of the soil (11) to be analyzed, wherein the impedance sensor comprises:
a first support element (12);
two conductive tracks (14) which are arranged on the first support element (12) but which are electrically insulated from this and from each other, at least one of which contains an electrically conductive, corrosion resistant polymer or composite material;
a control device (15a), which is configured to apply an AC voltage between the two conductive tracks (14), to vary its frequency over a predetermined frequency range, and during the course of this, during operation of the sensor device (1), when this is introduced into the soil (11) to be analyzed in such a way that the conductive tracks (14) are in electrical contact with the soil (11) to be analyzed, to detect an impedance spectrum of the soil (11) to be analyzed in response to the AC voltage applied to it via the conductive tracks (14) and to provide the impedance spectrum in the form of corresponding measurement data.

4. The sensor device (1) according to claim 3, wherein the first support element is electrically conductive, at least in an area which is covered by the conductive tracks (14), and the control device (15a) is further configured to apply a ground potential to this at least one area during the detection of the impedance spectrum of the soil (11) to be analyzed.

5. The sensor device (1) according to claim 3 or 4, wherein the predetermined frequency range includes the range from 100 Hz to 1 MHz.

6. The sensor device (1) according to any one of claims 3 to 5, wherein:
the first support element is constructed as a spike which, at least in part, is hollow, for at least partial introduction into the soil (11) to be analyzed,
wherein an insulation layer is applied to the surface of the spike, and on which, in turn, the two conductive tracks (14) are arranged; and
the control device (15a) is located in the interior of a hollow portion of the first support element (12).

7. The sensor device (1) according to any one of claims 3 to 6, wherein the sensor assembly comprises a temperature sensor (15c) for detecting a temperature of the soil (11) to be analyzed, wherein this, together with the impedance sensor, is constructed as an integrated impedance/temperature sensor assembly (3), which is configured to detect, simultaneously and in situ, an impedance spectrum as well as a temperature of the soil (11) to be analyzed and to make this available respectively in the form of corresponding measurement data.

8. The sensor device (1) according to any one of the preceding claims, wherein the sensor assembly comprises an absorption spectrometer assembly (5) for in situ detection of an absorption spectrum of the soil (11) to be analyzed, comprising:
at least two MEMS absorption spectrometers (26a, 26b), the spectral coverage of which differs at least for some portions of the electromagnetic spectrum, so that an absorption spectrum of the soil (11) to be analyzed can be detected cumulatively by the entirety of the MEMS absorption spectrometers (26a, 26b), which absorption spectrum has portions in the NIR range as well as in the VIS range and also in the UV range.

9. The sensor device (1) according to claim 8, wherein the absorption spectrometer assembly (5) further comprises a movable carrier (4) on which the absorption spectrometers are arranged in such a way that, when the carrier (24) is moved relative to a virtual measuring surface on which the soil (11) to be analyzed comes to rest during the measurement operation of the sensor device (1), they can spectrometrically measure an area of the soil (11) to be scanned by the absorption spectrometers in order to detect an absorption spectrum which is integrated over the area to be scanned.

10. The sensor device (1) according to claim 8 or 9, wherein the absorption spectrometer assembly (5) further comprises a movable shutter device (28) which is configured to temporarily move a screen into a space defined between the absorption spectrometers and the measuring surface, wherein a calibration reference (29) is arranged on the side of the screen which faces towards the absorption spectrometers, for the calibration of at least one of the absorption spectrometers.

11. The sensor device (1) according to any one of claims 8 to 10, wherein the absorption spectrometer assembly (5) further comprises an optical system (27) which, in a wavelength range corresponding to the absorption spectrum to be detected, is at least substantially optically transparent, which optical system (27) is arranged in the space between the absorption spectrometers and the measuring surface, in order to spatially separate these from each other;
wherein, on its side facing towards the measuring surface, the optical system (27) is provided with a hydrophilic nanocoating which improves the scratch protection.

12. The sensor device (1) according to any one of the preceding claims, wherein the sensor assembly comprises a potential measuring assembly (4) for in situ detection of an acidic or basic character of the soil (11) to be analyzed, comprising:
a second support element (17);
an electrolyte/metal reference electrode (19) which is arranged in or on the second support element;
a metal oxide electrode (21) which is arranged on a surface of the second support element (17), which surface is intended to contact the soil (11) to be analyzed during a measurement operation;
an ion diaphragm (20) which is arranged on the second support element between the metal oxide electrode (21) and the electrolyte/metal reference electrode (19) and which is in contact with the electrolyte/metal reference electrode (19);
a corrosion resistant calibration electrode (22) which is arranged on the surface of the second support element (17) provided for contacting the soil (11) to be analyzed and which corrosion resistant calibration electrode (22) is electrically insulated from the metal oxide electrode (21); and
a measuring device which is configured:
in order to determine a current state of the metal oxide electrode (21), to measure an electrical resistance arising between the calibration electrode (22) and the metal oxide electrode (21) and/or to measure an electrical capacitance arising therebetween when these two electrodes are each in contact with the soil (11) to be analyzed; and
in order to determine an acidic or a basic character of the soil (11) to be analyzed, to measure an electric potential difference arising between the reference electrode and the metal oxide electrode (21), taking into account a measurement calibration previously determined on the basis of the determined current state of the metal oxide electrode (21), when these two electrodes are each in contact with the soil (11) to be analyzed.

13. The sensor device (1) according to claim 12, wherein the calibration electrode (22) is made of a material that contains an electrically conductive and corrosion resistant polymer or composite material.

14. The sensor device (1) according to claim 12 or 13 wherein the second support element is constructed as a spike for at least partial introduction into the soil (11) to be analyzed, wherein an insulating layer is applied to the surface of the spike, on which insulating layer the metal oxide electrode (21), the ion diaphragm (20) and/or the calibration electrode (22) are arranged.

15. The sensor device (1) according to any one of the preceding claims, further comprising a communication device (8) for the transmission of detected measurement data to a counterpart which is external with respect to the sensor device (1), for evaluation.

16. The sensor device (1) according to claim 15, wherein the communication device (8) is configured to transmit the measurement data wirelessly by means of communication on the basis of LoRa radio technology and/or NarrowBand Internet of Things, NB-IoT, radio technology.

17. The sensor device (1) according to claim 15 or 16, further comprising a secure storage device for storing, protected against unauthorized access, a unique device identification of the sensor device (1) and/or at least a cryptographic key for encrypting measurement data and/or metadata transmitted by means of the communication device (8).

18. The sensor device (1) according to any one of claims 15 to 17, wherein the communication device (8) is further configured to write, into a block chain acting as an external counterpart, measurement data and/or metadata to be transmitted, or to cause another external counterpart to write, into a block chain, the measurement data and/or metadata transmitted to it.

19. The sensor device (1) according to claim 18, wherein the sensor device (1) is configured to carry out an authentication of a user of the sensor device (1) and to allow the transmission of measurement data and/or metadata to an external counterpart only if the authentication has been successful.

20. The sensor device (1) according to any one of the preceding claims, further comprising a position determination device (7) for determining a current position of the sensor device (1) and to provide corresponding metadata characterizing the position.

21. A computer-implemented method for soil analysis, comprising:
receiving measurement data relating to the following soil properties of a soil (11) to be analyzed:
(a) an impedance spectrum (Z(ω)), indicating the alternating current resistance of a portion of soil (11) to be analyzed as a function of the frequency (ω) of an alternating measuring voltage which is applied to the portion of soil,
(b) a temperature,
(c) an absorption spectrum in a spectral range which extends from NIR to UV, NIR-VIS-UV
determining at least one of the soil properties or at least one soil property derived therefrom on the basis of a combination of the received measurement data by means of data fusion in order to obtain a respective measurement result for the at least one soil property to be determined.

22. The computer-implemented method according to claim 21, further comprising:
receiving measurement data relating to the following soil property of the soil (11) to be analyzed: an acidic or basic character.

23. The computer-implemented method according to claim 21 or 22, wherein the measurement data is detected by a sensor device (1) according to any one of claims 1 to 20.

24. The computer-implemented method according to any one of claims 21 to 23, wherein the method is carried out in at least one central node (33) of a network, which at least one central node (33), in order to receive the respective measurement data, is configured to be in communication connection (31) with a plurality of sensor devices (1) for detecting the respective measurement data.

25. A computer program which is configured, when it is run on a processor platform, to carry out the computer-implemented method according to any one of claims 21 to 24.

26. A device (33) for soil analysis, wherein the device is arranged to carry out the computer-implemented method according to any one of claims 21 to 24.

27. The device (1, 33) according to claim 26, comprising a sensor device (1) according to any one of claims 1 to 20 for detecting the measurement data.

## Revendications

1. Dispositif de détection (1) pour l'analyse de sol in situ, présentant :
un ensemble de capteur avec un ou plusieurs capteurs, qui sont configurés individuellement ou de manière cumulative pour la mesure in situ simultanée des propriétés de sol suivantes d'un sol (11) à analyser et pour la fourniture de données de mesure respectives correspondantes :
(a) le spectre d'impédance ;
(b) la température ; et
(c) le spectre d'absorption dans un domaine spectral NIR-VIS-UV allant des NIR aux UV ;
dans lequel :
la distance entre respectivement deux des capteurs de l'ensemble de capteur définie par rapport à leurs détecteurs de grandeur de mesure respectifs ne dépasse pas une valeur de 10 cm ;
l'ensemble de capteur est configuré pour la mesure in situ du spectre d'impédance (Z(ω)) pour mesurer une résistance en alternatif d'une partie de sol à mesurer en fonction de la fréquence (ω) d'une tension alternative de mesure appliquée à la partie de sol ; et
l'ensemble de capteur est configuré pour la mesure in situ du spectre d'absorption pour enregistrer un rayonnement réfléchi sur la surface de sol du sol (11) à analyser dans le domaine spectral cité.

2. Dispositif de détection (1) selon la revendication 1, dans lequel l'ensemble de capteur présente en outre un ou plusieurs capteurs, qui sont configurés individuellement ou de manière cumulative pour mettre en œuvre une mesure in situ simultanée avec les autres mesures d'un caractère acide ou basique du sol (11) à analyser et pour fournir des données de mesure correspondantes.

3. Dispositif de détection (1) selon la revendication 1 ou 2, dans lequel l'ensemble de capteur contient pour l'acquisition in situ d'un spectre d'impédance du sol (11) à analyser un capteur d'impédance, qui présente :
un premier élément de support (12) ;
deux tracés conducteurs (14) disposés sur le premier élément de support (12) mais de manière isolée électriquement de celui-ci et les uns des autres, dont au moins un contient un matériau polymère ou composite électriquement conducteur résistant à la corrosion ;
un dispositif de commande (15a), qui est configuré pour appliquer entre les deux tracés conducteurs (14) une tension alternative, pour modifier la fréquence de celle-ci par-dessus une plage de fréquences prédéfinie, et ce faisant pour acquérir lors du fonctionnement du dispositif de détection (1), lorsque celui-ci est introduit dans le sol (11) à analyser de sorte que les tracés conducteurs (14) sont en contact électrique avec celui-ci, pour acquérir un spectre d'impédance du sol (11) à analyser en réponse à la tension alternative appliquée sur celui-ci par l'intermédiaire des tracés conducteurs (14) et pour le fournir sous forme de données de mesure correspondantes.

4. Dispositif de détection (1) selon la revendication 3, dans lequel le premier élément de support est électriquement conducteur au moins dans une zone couverte par les tracés conducteurs (14) et le dispositif de commande (15a) est en outre configuré pour, pendant l'acquisition du spectre d'impédance du sol (11) à analyser, placer le potentiel électrique de cette au moins une zone à un potentiel de masse.

5. Dispositif de détection (1) selon la revendication 3 ou 4, dans lequel la plage de fréquences prédéfinie renferme la plage de 100 Hz à 1 MHz.

6. Dispositif de détection (1) selon l'une quelconque des revendications 3 à 5, dans lequel :
le premier élément de support est réalisé en tant que mandrin creux au moins sur certaines parties pour être introduit au moins sur certaines parties dans le sol (11) à analyser,
dans lequel une couche isolante est appliquée sur la surface du mandrin, et les deux tracés conducteurs (14) sont disposés à leur tour sur celle-ci ; et
le dispositif de commande (15a) est disposé à l'intérieur d'une partie creuse du premier élément de support (12).

7. Dispositif de détection (1) selon l'une quelconque des revendications 3 à 6, dans lequel l'ensemble de capteur contient pour l'acquisition d'une température du sol (11) à analyser un capteur de température (15c), dans lequel celui-ci est réalisé conjointement avec le capteur d'impédance comme un ensemble de capteur d'impédance/de température (3) intégré, qui est configuré pour acquérir simultanément et in situ aussi bien un spectre d'impédance qu'une température du sol (11) à analyser et pour les fournir respectivement sous forme de données de mesure correspondantes.

8. Dispositif de détection (1) selon l'une quelconque des revendications précédentes, dans lequel l'ensemble de capteur contient pour l'acquisition in situ d'un spectre d'absorption du sol (11) à analyser un ensemble de spectromètre d'absorption (5), qui présente :
une source (25) pour un rayonnement électromagnétique, dont le rayonnement couvre le domaine spectral NIR-VIS-UV cité allant de NIR aux UV ;
au moins deux spectres d'absorption MEMS (26a, 26b), dont la couverture spectrale se différencie au moins pour des zones partielles du spectre électromagnétique, de sorte qu'un spectre d'absorption du sol (11) à analyser peut être acquis de manière cumulative par la totalité du spectre d'absorption MEMS (26a, 26b), lequel présente aussi bien des fractions dans le domaine NIR que dans le domaine VIS que dans le domaine UV.

9. Dispositif de détection (1) selon la revendication 8, dans lequel l'ensemble de spectromètre d'absorption (5) présente en outre un support (4) mobile, sur lequel les spectres d'absorption sont disposés, de sorte que lors d'un mouvement du support (24) par rapport à une surface de mesure virtuelle, sur laquelle le sol (11) à analyser vient se situer lorsque le dispositif de détection (1) est en mode mesure, ils peuvent mesurer de manière spectrométrique une zone du sol (11) destinée à être balayée par les spectres d'absorption, afin d'acquérir un spectre d'absorption intégré sur toute l'étendue de la zone à balayer.

10. Dispositif de détection (1) selon la revendication 8 ou 9, dans lequel l'ensemble de spectromètre d'absorption (5) présente en outre un dispositif d'obturateur (28) mobile, qui est configuré pour rentrer temporairement un diaphragme dans une zone spatiale définie entre les spectres d'absorption et la surface de mesure, dans lequel une référence d'étalonnage (29) pour l'étalonnage d'au moins un des spectres d'absorption est disposée sur la face du diaphragme tournée vers les spectres d'absorption.

11. Dispositif de détection (1) selon l'une quelconque des revendications 8 à 10, dans lequel l'ensemble de spectromètre d'absorption (5) présente en outre une optique (27) au moins sensiblement optiquement transparente, dans une plage de longueurs d'onde correspondant au spectre d'absorption à enregistrer, qui est disposée dans la zone spatiale entre les spectres d'absorption et la surface de mesure, afin de séparer ceux-ci spatialement les uns des autres ;
dans lequel l'optique (27) est pourvue sur sa face tournée vers la surface de mesure d'un nanorevêtement hydrophile et améliorant la protection contre les rayures.

12. Dispositif de détection (1) selon l'une quelconque des revendications précédentes, dans lequel l'ensemble de capteur pour l'acquisition in situ d'un caractère acide ou basique du sol (11) à analyser contient un ensemble de mesure de potentiel (4), qui présente :
un deuxième élément de support (17) ;
une électrode de référence métallique/électrolytique (19) disposée dans ou sur le deuxième élément de support ;
une électrode en oxyde métallique (21) disposée sur une surface du deuxième élément de support (17) prévue pour être en contact avec le sol (11) à analyser lors du mode mesure ;
un diaphragme ionique (20) disposé sur le deuxième élément de support entre l'électrode en oxyde métallique (21) et l'électrode de référence métallique/électrolytique (19) et en contact avec l'électrode de référence métallique/électrolytique (19) ;
une électrode d'étalonnage (22) résistante à la corrosion disposée sur la surface du deuxième élément de support (17) prévue pour être en contact avec le sol (11) à analyser et électriquement isolée de l'électrode en oxyde métallique (21) ; et
un système de mesure, qui est configuré :
pour la détermination d'un état actuel de l'électrode en oxyde métallique (21) pour mesurer une résistance électrique apparaissant entre l'électrode d'étalonnage (22) et l'électrode en oxyde métallique (21) et/ou une capacité électrique apparaissant entre celles-ci, lorsque ces deux électrodes sont respectivement en contact avec le sol (11) à analyser ; et
pour la détermination d'un caractère acide ou basique du sol (11) à analyser pour mesurer une différence de potentiel électrique apparaissant entre l'électrode de référence et l'électrode en oxyde métallique (21) avec prise en compte d'un étalonnage de mesure préalablement établi sur la base de l'état actuel déterminé de l'électrode en oxyde métallique (21), lorsque ces deux électrodes sont respectivement en contact avec le sol (11) à analyser.

13. Dispositif de détection (1) selon la revendication 12, dans lequel l'électrode d'étalonnage (22) est fabriquée à partir d'une matière qui contient un matériau polymère ou composite électriquement conducteur et résistant à la corrosion.

14. Dispositif de détection (1) selon la revendication 12 ou 13, dans lequel le deuxième élément de support est réalisé en tant que mandrin à introduire au moins sur certaines parties dans le sol (11) à analyser, dans lequel une couche isolante, sur laquelle l'électrode en oxyde métallique (21), le diaphragme ionique (20) et/ou l'électrode d'étalonnage (22) sont disposés, est appliquée sur la surface du mandrin.

15. Dispositif de détection (1) selon l'une quelconque des revendications précédentes, présentant en outre un dispositif de communication (8) pour la transmission de données de mesure acquises pour l'évaluation de celles-ci à une face opposée externe par rapport au dispositif de détection (1).

16. Dispositif de détection (1) selon la revendication 15, dans lequel le dispositif de communication (8) est configuré pour transmettre les données de mesure sans fil au moyen d'une communication basée sur la technologie radio LoRa et/ou la technologie radio NarrowBand-Internet of Things, NB-loT.

17. Dispositif de détection (1) selon la revendication 15 ou 16, présentant en outre un dispositif de stockage sécurisé pour le dépôt protégé contre un accès non autorisé d'une identification d'appareil univoque du dispositif de détection (1) et/ou d'au moins une clé cryptographique pour le chiffrement de données de mesure et/ou métadonnées transmises au moyen du dispositif de communication (8).

18. Dispositif de détection (1) selon l'une quelconque des revendications 15 à 17, dans lequel le dispositif de communication (8) est configuré en outre pour écrire des données de mesure et/ou métadonnées à transmettre dans une chaîne de blocs apparaissant en tant que face opposée externe ou pour amener une autre face opposée externe à écrire les données de mesure et/ou métadonnées transmises à celle-ci dans une chaîne de blocs.

19. Dispositif de détection (1) selon la revendication 18, dans lequel le dispositif de détection (1) est configuré pour réaliser une authentification d'un utilisateur du dispositif de détection (1) et pour ne permettre une transmission des données de mesure et/ou métadonnées à une face opposée externe que lorsque l'authentification s'est passée avec succès.

20. Dispositif de détection (1) selon l'une quelconque des revendications précédentes, présentant en outre un dispositif de détermination de position (7) pour la détermination d'une position actuelle du dispositif de détection (1) et pour la fourniture de métadonnées correspondantes identifiant la position.

21. Procédé implémenté sur ordinateur pour l'analyse de sol, présentant :
la réception de données de mesure concernant les propriétés de sol suivantes d'un sol (11) à analyser, dans lequel les données de mesure représentent ce qui suit :
(a) un spectre d'impédance (Z(ω)), qui indique la résistance en alternatif d'une partie de sol à mesurer en fonction de la fréquence (ω) d'une tension an alternatif appliquée sur la partie de sol,
(b) une température,
(c) un spectre d'absorption dans un domaine spectral NIR-VIS-UV allant de NIR aux UV ; et
la détermination d'au moins une des propriétés de sol ou d'au moins une propriété de sol déduite de celles-ci sur la base d'une combinaison des données de mesure reçues au moyen d'une fusion de données pour l'obtention d'un résultat de mesure respectif pour la au moins une propriété de sol à déterminer.

22. Procédé implémenté sur ordinateur selon la revendication 21, présentant en outre :
la réception de données de mesure par rapport à la propriété de sol suivante du sol (11) à analyser : caractère acide ou basique.

23. Procédé implémenté sur ordinateur selon la revendication 21 ou 22, dans lequel les données de mesure sont acquises par un dispositif de détection (1) selon l'une quelconque des revendications 1 à 20.

24. Procédé implémenté sur ordinateur selon l'une quelconque des revendications 21 à 23, dans lequel le procédé est réalisé dans au moins un nœud central (33) d'un réseau, qui est configuré pour la réception des données de mesure respectives pour être en liaison de communication (31) avec une pluralité de dispositifs de détection (1) pour l'acquisition des données de mesure respectives.

25. Programme informatique, qui est configuré, lors de son exécution sur une plateforme de processeur, pour mettre en œuvre le procédé implémenté sur ordinateur selon l'une quelconque des revendications 21 à 24.

26. Dispositif (33) pour l'analyse de sol, dans lequel le dispositif est conçu pour mettre en œuvre le procédé implémenté sur ordinateur selon l'une quelconque des revendications 21 à 24.

27. Dispositif (1, 33) selon la revendication 26, présentant un dispositif de détection (1) selon l'une quelconque des revendications 1 à 20 pour l'acquisition des données de mesure.
